(19) Europäisches Patentamt
European Patent Office
Office européen des brevets

(11) EP 4 364 790 A1

(12) EUROPEAN PATENT APPLICATION

(43) Date of publication:
08.05.2024 Bulletin 2024/19

(51) International Patent Classification (IPC):
A61N 1/36 (2006.01)

(21) Application number: 22204926.4

(52) Cooperative Patent Classification (CPC):
A61N 1/3605

(22) Date of filing: 01.11.2022

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(71) Applicant: ECOLE POLYTECHNIQUE FEDERALE DE LAUSANNE (EPFL)
1015 Lausanne (CH)

(72) Inventors:
• ROMENI, Simone
74100 Ambilly (FR)
• MICERA, Silvestro
1204 Genève (CH)

(74) Representative: Weihs, Bruno Konrad et al
André Roland SA
IP Attorneys & Services
Avenue Collonges 21
1004 Lausanne (CH)

(54) **A METHOD AND SYSTEM FOR OPTIMIZING DESIGN, IMPLANTATION, AND STIMULATION PROTOCOLS OF ELECTRODES STIMULATING THE NERVOUS SYSTEM**

(57) A method for optimizing at least one of a geometry, an implantation procedure, and/or stimulation protocol of one or more electrodes for an electrical stimulation of a target structure in a nervous system of a living being by a computer device, the method comprising the steps of providing a morphology of the target structure, performing a geometric optimization of the one or more electrodes based on (i) a geometric parametrization of the one or more electrodes and the morphology of the target structure, and (ii) an objective function evaluating a quality of the one or more electrodes in relationship to the target structure, finite element modelling (FEM) of the one or more electrodes implanted in the target structure according to a geometry found during the step of performing the geometric optimization to generate electric potential maps, interpolating the electric potential maps to potential fiber node locations of the target structure to build a lead field matrix (LFM), and biophysical modeling of fiber stimulation to create a dataset of biophysical model responses based on the LFM and a first subset of potential stimulation protocols.

FIG. 4

EP 4 364 790 A1

**Description**

FIELD OF THE INVENTION

The present invention is directed to the field of stimulation of the nervous system of a living being by the use of electrodes, more preferably directed to a method or system to optimize or improve upon the stimulation protocols that are used for the simulation of the nervous systems.

BACKGROUND

Electrical stimulation of the nervous system through implantable electrodes has been employed in the past decades to treat neuropathic pain 1, provide sensory feedback to robotic limbs 2,3, restore walking in paralyzed patients 4, treat the tremor associated to Parkinson's disease 5, among others. Computational models have been thoroughly used in the study of neuromodulation, as they allow to investigate the various aspects of electrical stimulation of the nervous system and could be used to improve stimulation systems employing the gained insight. In particular, hybrid models (HM), have been widely used to explain the fundamental mechanisms of peripheral nerve 6, spinal cord 7, and deep brain stimulation 8. Nonetheless, because of their high computational complexity, they have not been used to perform a true optimization of the design and operation of implantable electrodes.

Some attempts have been made to either analyse systematically the effect of different choices of geometrical parameters for extraneural electrodes for peripheral nerve stimulation 9, or to set up 10 and use 11 of lightweight methods to estimate the activation of nerve fibers thus allowing to evaluate a high number of stimulation protocols. Both 9 and 11 are believed to be the closest prior art.

Nonetheless, as we already mentioned, their approaches do not include a proper optimization routine, but rather evaluate the performance of the geometry / stimulation protocol on a uniform grid of parameter values, to finally choose the best set of parameter values. Additionally, they do not incorporate structural or functional morphology information, their approach is not generalizable to nonlinear behaviours nor to branched neural elements, and they do not provide any way to reduce the weight of the finite element modelling step, which here we propose to substitute with a machine learning-based surrogate-model. Therefore, according to these deficiencies of the state of the art, substantially improved methods and systems for optimizing at least one of a geometry, an implantation procedure, and/or stimulation protocol of one or more electrodes for an electrical stimulation are desirable.

SUMMARY OF THE INVENTION

According to an aspect of the present invention, a method for optimizing at least one of a geometry, an implantation procedure, and/or stimulation protocol of one or more electrodes for an electrical stimulation of a target structure in a nervous system of a living being by a computer device is provided. Preferably, the method includes the steps of providing a morphology of the target structure, performing a geometric optimization of the one or more electrodes based on (i) a geometric parametrization of the one or more electrodes and the morphology of the target structure, and (ii) an objective function evaluating a quality of the one or more electrodes in relationship to the target structure, finite element modelling (FEM) of the one or more electrodes implanted in the target structure according to a geometry found during the step of performing the geometric optimization to generate electric potential maps, and interpolating the electric potential maps to potential fiber node locations of the target structure to build a lead field matrix (LFM).

Moreover, preferably, the method further comprises the steps of biophysical modeling of stimulation of neural elements populating the neural structure to create a dataset of biophysical model responses based on the LFM and a first subset of potential stimulation protocols, fitting responses of a model-based surrogate model or training a machine-learning-based surrogate model of the neural elements of the target structure to the electrical stimulation administered by the one or more electrodes based on the dataset of the biophysical model responses, estimating a functional topography of the target structure using a set of recordings or stimulation protocols from the one or more electrodes, the step of estimating being performed after implanting the one or more electrodes, performing an optimization of a second subset of the potential stimulation protocols based on (i) the surrogate-model of a response of the neural element to stimulation, (ii) the morphology of the target structure, and (iii) an objective function quantifying an effect of the second subset of potential stimulation protocols to generate a set of candidate optimal stimulation protocols, and evaluating the set of candidate optimal stimulation protocols by applying the set of candidate optimal stimulation protocols to the biophysical modeling to determine an optimum stimulation protocol for the target structure.

In a preferred embodiment, the method further comprises the step of obtaining the morphology of the target structure by at least one of magnetic resonance imaging (MRI) or electrosonography.

In a further preferred embodiment, the target structure includes a nerve, and the morphology includes a subdivision of a tridimensional structure of a nerve into a plurality of subregions targeting different functions.

In a further preferred embodiment, the subregions targeting different functions include subregions that connect to different muscles, organs, or a combination thereof.

In a further preferred embodiment, the morphology includes information on a fascicular morphology of the nerve.

In a further preferred embodiment, the target structure includes a brain, and the morphology includes a subdivision of a tridimensional structure of the brain into a plurality of subregions, the subregions performing different functions.

In a further preferred embodiment, the target structure includes a spinal cord root, and the morphology includes a subdivision of a tridimensional structure of the spinal cord root into a different rootlets.

In a further preferred embodiment, the performing the geometric optimization of the one or more electrodes, the objective function includes a second surrogate-model of the FEM of the one or more electrodes and a stimulation protocol optimization.

In a further preferred embodiment, the second surrogate-model of the FEM includes an encoder-decoder with a U-Net architecture accepting as input a set of two 3D tensors that define the morphology of the target structure and the first subset of potential stimulation protocols, respectively, and providing an output including a 3D tensor including a value of an extracellular potential at different locations of the target structure.

In a further preferred embodiment, the step of estimating the functional topography is performed using a functional MRI.

In a further preferred embodiment, the step of estimating the functional topography is performed using information from recruitment curves obtained by monopolar electrical stimulation.

In a further preferred embodiment, the step of fitting further includes building a surrogate-model including a binary activation classifier that predicts whether neural units are activated by an applied stimulation protocol.

In a further preferred embodiment, the target structure includes nerve fibers, and the binary activation classifier includes a multilayer perceptron, a support vector machine, a random forest, or a Gaussian process classifier, or a combination thereof, where each of the aforementioned binary activation classifier receive as input data a timely evolution of the extracellular potential appliced to the nerve fibers and a diameter of the nerve fibers.

In a further preferred embodiment, the target structure includes complex neurons, and the binary activation classifier includes a graph neural network that operates graph classification using a topology of the complex neurons, including information about interconnections between soma, axon, and dendrites, and the extracellular potential applied to the different sections of the complex neurons, and their corresponding morphological features as nodal features.

In a further preferred embodiment, the step of building the surrogate-model is based on a neural response to an electric stimulation of the one or more electrodes, the surrogate model including a single-output regressor that predicts a firing rate produced in neural units under the applied electric stimulation.

In a further preferred embodiment, the step of building the surrogate-model is based on a neural response to an electric stmulation of the one or more electrodes, the surrogate model including a sequence-to-sequence predictor that predicts a timely evolution of a membrane potential produced in the neural units under the applied electric stimulation.

In a further preferred embodiment, the target structure includes a target nerve, and wherein in the step of performing the geometric optimization, the objective function includes a first function for minimizing an average distance between nerve fascicles of the target nerve and an implant site of the one or more electrodes.

In a further preferred embodiment, in the step of performing the geometric optimization, the objective function includes a second function for maximizing a number of target nerve fascicles of the target nerve.

In a further preferred embodiment, the target structure includes a target nerve, and wherein in the step of biophysical modeling, conductance-based cable models of nerve fibers of the target nerve are used.

In a further preferred embodiment, in the step of biophysical modeling, conductance-based compartmental models of neurons are used.

In a further preferred embodiment, the set of candidate optimal stimulation protocols includes a time-series representing the electric current waveforms injected by the stimulating sites of the one or more electrodes.

In a further preferred embodiment, the stimulation protocols have a fixed waveform depending on a number of parameters and the step of performing the optimization of the applied subset of the stimulation protocols produced values of the parameters of the fixed wavefrom, for each stimulating site of the one more more electrodes.

In a further preferred embodiment, the model-based surrogate-model includes an activating function formalism.

The above and other objects, features and advantages of the present invention and the manner of realizing them will become more apparent, and the invention itself will best be understood from a study of the following description with reference to the attached drawings showing some preferred embodiments of the invention.

BRIEF DESCRIPTION OF THE SEVERAL VIEWS OF THE DRAWNGS

The accompanying drawings, which are incorporated herein and constitute part of this specification, illustrate the presently preferred embodiments of the invention, and together with the general description given above and the detailed description given below, serve to explain features of the invention.

FIG. 1 shows a simplified flowchart showing a brief overview of the method for optimizing at least one of a geometry, an implantation procedure, and/or stimulation protocol of one or more electrodes for an electrical stimulation of a target structure in a nervous system of a living being by a computer device;

FIG. 2 shows features and set-ups for steps of the method illustrated in FIG. 1;

FIG. 3 shows cadaveric histological sections used for the analysis of one aspect of the present invention;

FIG. 4 shows a more detailed flow chart of the method for optimizing at least one of a geometry, an implantation procedure, and/or stimulation protocol of one or more electrodes, according to an aspect of the present invention, illustrating a proposed opzimization workflow;

FIGs. 5A to 5D shows different graphs that illustrate the dimensioning and choice of the number of implanted electrodes;

FIGs. 6A to 6D show multiple insertion optimization routines on the same section;

FIGs. 7A to 7C show different representations of the characterization of candidate optimal implant geometries;

FIGs. 8A to 8C show resuls of the candicate optimal stimulation protocol evaluation;

FIGs. 9A to 9C show stimulation protocol optimization in different conditions;

FIGs. 10A to 10E illustrate stimulation protocol optimization for different selectivity measures;

FIG. 11 shows a table representing the occurance of the couples of electrode insertions that occurred most frequently together;

FIG. 12 shows a table representing the expected classification accuracies and the number of samples in the balanced dataset corresponding the trained classifiers;

FIG. 13 shows a 2D UNet encoder-decoder network architecture with a number of trainable weights for each layer and for the whole network, which has been employed as a surrogate model of the FEM;

FIG. 14 shows the performance of a trained surrogate model of the FEM on a subset of the test set;

FIG. 15 shows the performance of a trained MLP surrogate model for the prediction of nerve fiber firing rates under periodic stimulation protocols on a subset of the test set;

FIG. 16 shows a convolutional neural network architecture with the number of trainable weights for each layer and for the whole networks, which has been employed as a surrogate model of the neural element response to stimulation in the case of aperiodic stimulation protocols;

FIG. 17 shows a confusion matrix for the classification of the activation of nerve fibers under aperiodic stimulation on an employed test set;

FIGs. 18A to 18E show the different phases of motor fiber cluster localization in the section of a peripheral nerve using stimulation; and

FIGs. 19A to 19E show the results of the stimulation optimization to maximize the stimulation selectivity of each fiber group in FIGs. 18A to 18E, using the localization shown in FIG. 18A to 18E to establish the location of the groups.

## DETAILED DESCRIPTION OF THE SEVERAL EMBODIMENTS

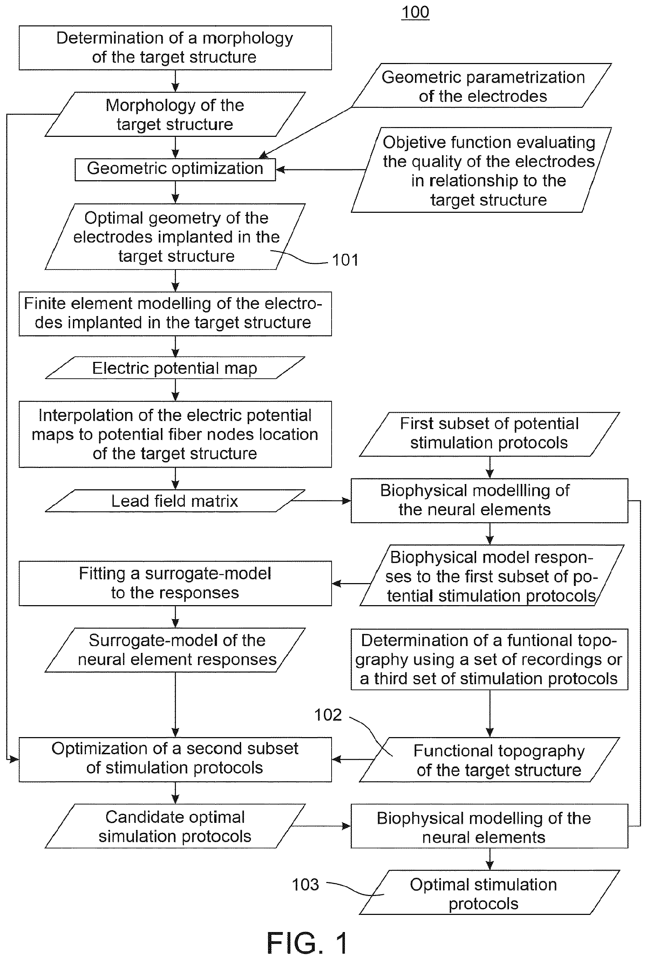

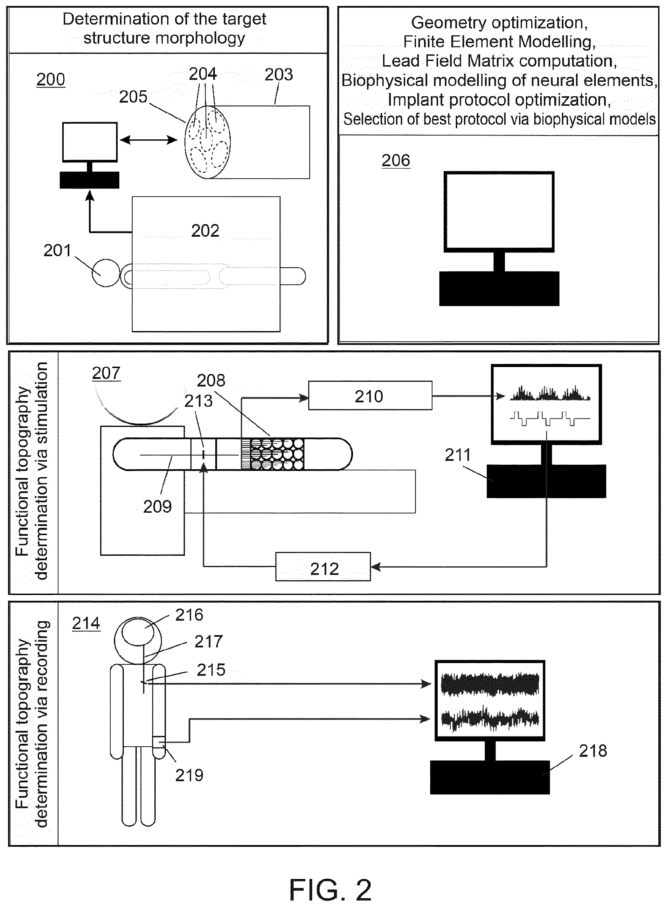

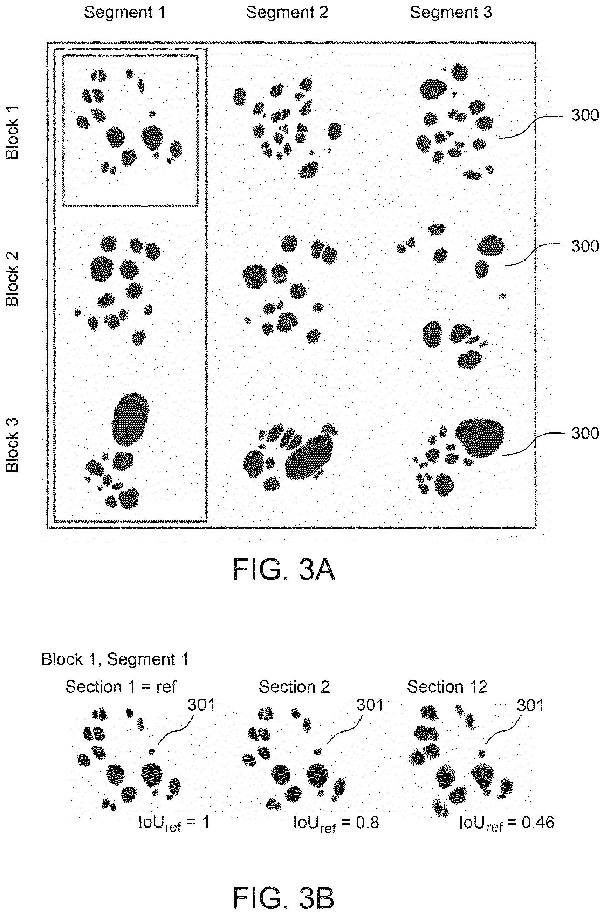

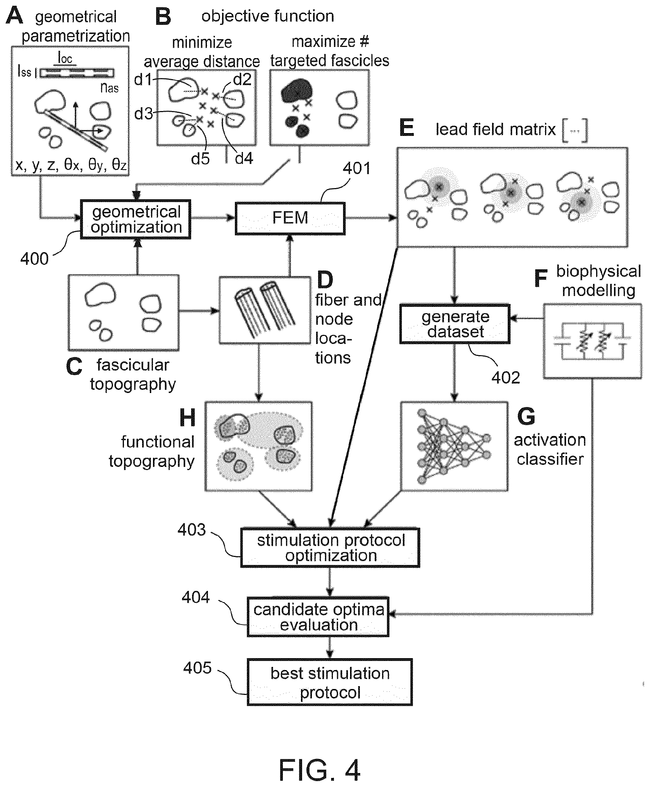

**FIG. 1** shows a simplified flowchart **100** showing a brief overview of the method for optimizing at least one of a geometry **101,** an implantation procedure **102,** and/or stimulation protocol **103** of one or more electrodes for an electrical stimulation of a target structure in a nervous system of a living being by a computer device (target structure, nervous system and computer device not illustrated in **FIG. 1). FIG. 2** shows features and set-ups for steps of the method illustrated in **FIG. 1,** comprising a set-up **200** for a determination of the target structure morphology, which shows a patient **201** being submitted to a magnetic resonance imaging procedure **202** which result in the determination of the **structure** morphology of a nerve **203,** revealing nerve fascicles **204** in a nerve cross-section **205. FIG. 2** further comprises a box **206** illustrating the process of geometry optimization, Finite Element Modelling, Lead Field Matrix computation, Biophysical modelling of neural elements, Implant protocol optimization, and Selection of best protocol via biophysical models. Box **207** contains an illustration of a functional topography determination via stimulation with an EMG electrode array **208** configured to deliver a signal from a median nerve **209** to an EMG aquisition system **210.** Computer **211** receives this signal and outputs a control to a neural stimulator **212** which in turn sends a stimulation signal to a multielectrode implant **213. FIG. 2** further comprises a box **214** illustrating a functional topography determination via recording in which a multielectrode implant **215** is configured to measure a signal from brain **216** at vagus nerve **217** and input this to computer **218.** The computer **218** also inputs a signal from a heart rate variability sensor **219** of a patient. **FIGs. 3A** and **3B** shows cadaveric histological sections **300, 301** used for the analysis of one aspect of the present invention as illustrated for example in **FIGs. 4** to **12.**

According to an aspect of the present invention, with the herein presented method and system an optimized workflow can be provided that can guide both the pre-surgical planning and the determination of maximally selective multisite stimulation protocols for implants, the implants including one or more intraneural electrodes, and the results have been characterized by its performance *in silico.* Basically, a method is herewith proposed that can serve as guidelines to use effectively hybrid models (HMs) for the optimization of peripheral nerve personalized neuroprostheses for sensory and motor function restoration, and for bioelectronic medicine.

A simplified block diagram of the proposed method and workflow is shown in **FIG. 4.** More precisely, **FIG. 4** shows a more detailed flow chart of the method for optimizing at least one of a geometry, an implantation procedure, and/or stimulation protocol of one or more electrodes, according to an aspect of the present invention, illustrating a proposed opzimization workflow, with element **A** showing parametrization of the electrode geometry and insertion. Here, the parametrization of a TIME-like electrode is shown ($n_{as}$: number of active sites per electrode face; $\ell_{cc}$: distance between active sites on the same electrode face; $\ell_{ff}$: distance between electrode faces; ($x,y,z$): location of the electrode in the nerve reference frame; ($\theta_x, \theta_y, \theta_z$): orientation of the electrode in the nerve reference frame), element **B** representing an Objective function for implant geometry optimization. Here, we show the minimization of the average minimum distance between fascicles and active sites (m-AMD), and the maximization of the number of pierced fascicles (M-NPF). In m-AMD, $d_x$ indicates the distance between each fascicle (6 fascicles here) and the closest active site. In M-NPF, the pierced fascicles are in shaded in red, and element **C** representing Fascicular topography of the nerve (fascicle boundary polylines in the nerve reference frame). Elements **A, B,** and **C** are input to the geometrical optimization routine **400,** which produces the optimal implant geometry input to the finite element modelling (FEM) **401.** Element **D** shows a determination of fiber locations in the nerve section and of fiber nodes along the nerve fiber path. It is constrained by the fascicular topography of the nerve (fibers should be inside fascicles). Element **E** shows the characterization of the forward electrical problem by means of a lead field matrix, whose components correspond to the electric potential map produced by a unit-amplitude single-site stimulation. The optimal implant geometry and fiber node locations. The results of element **D** are input to FEM **401** to produce element **E.** Element **F** shows biophysical modelling of fiber stimulation. Here, conductance-based cable models of nerve fibers are used. Elements **E** and **F** are used to generate a dataset **402** associating fiber stimulation and consequent activation. (Element **G**) Activation classifier (here, a multilayer perceptron (MLP) is shown) trained on the generated fiber-stimulation-activation data. Element **H** represents Nerve functional topography (assignment of nerve fibers to functional groups). Here, each grey scale represents the region of the nerve that targets a specific function. Fibers in a given region target the corresponding function. The results of Elements **E, G,** and **H** are input to stimulation protocol optimization, which produces a set of candidate optimal stimulation protocols **403** (here, protocols that maximize group-wise selectivity). Candidate optimal stimulation protocols **404** are evaluated using the biophysically detailed model represented by element **F** to establish the true optimal stimulation protocol **405.**

The first conceptual block of the workflow is implant geometry optimization, which starting from the morphology of the target nerve, provided in the form of its fascicular topography, see section Element **A** of **FIG. 4,** and a parametrization of the electrode geometry and insertion in the nerve is shown in section Element **B** of **FIG. 4,** finding the optimal implant geometry **400** and implant procedure according to a predetermined objective function is shown in section Element **C** of **FIG. 4.** The potential locations of nerve fibers in the nerve section and of fiber nodes along the fibers are sampled, as illustrated in section Element **D** of **FIG. 4.** Moreover, the FEM model **401** of the nerve section and the inserted optimal implant is built, and a simulation is performed for each active site, with the given active site injecting a unit current and all other electrode sites switched off. The electric potential maps resulting from these simulations are interpolated to fiber node locations and are collected in the so-called lead field matrix (LFM) of the problem, see section Element **E** of **FIG. 4.**

Using the LFM, the extracellular potential at the fiber nodes for any multi-site stimulation protocol can be generated, which can be applied to a biophysically detailed model of a nerve fiber, see section Element **F** of **FIG. 4,** to determine if the fiber is active under that protocol. Thus, the large dataset **402** of extracellular potential distributions and corresponding fiber activations is generated, and used to train a multilayer perceptron (MLP) binary classifier, shown in section Element **G** of **FIG. 4.** The MLP is used in the stimulation protocol optimization **403** to predict the activation of the whole fiber population for each candidate stimulation protocol **404.** Fiber activations are then converted to recruitment and selectivity values for the different fiber groups by taking into account the functional topography of the nerve, illustrated in section Element **H** of **FIG. 4.** Finally, candidate optimal stimulation protocols **405** are evaluated by applying them to the biophysically detailed neural population in the nerve section.

These routines were tested on histological sections taken from the cadaveric samples of the human median nerve described in detail as shown in the reference 12. These sections have been divided into nerve segments and blocks, where a block contains several segments, and for each segment we have identified a representative mask and have ordered the remaining masks in order of increasing dissimilarity from the representative mask.

Both implant geometry and stimulation protocol optimization were carried on by particle swarm optimization (PSO). Implant geometry optimization was employed to determine the optimal insertion for each implanted electrode and, for the first experiment, the optimal inter-site distance with respect to one of two objective functions that we introduced. The first objective is the minimization of the average distance between each fascicle and the closest electrode site (m-AMD), and the second is the maximization of the number of fascicles having at least one stimulating site (M-NPF). Stimulation protocol optimization was employed to determine which sites are active and with which currents they are stimulating, with the aim of maximizing the selectivity of the stimulation of a given fiber functional group. In each nerve section, we distributed twelve (12) fiber groups, each corresponding to a different muscle innervated by the human median nerve as shown in reference 12, according to the criteria exposed the methods described below. The *PSO parameters employed* in the two cases are listed in a dedicated paragraph herein below.

For the herein discussed transverse intrafascicular multichannel electrodes (TIMEs), such parameters are the number of active sites per face ($n_{as}$). the distance between active sites on the same face ($\ell_{cc}$),the distance between the two faces ($\ell_{ff}$), and the exemplary six (6) geometrical parameters defining the pose of the electrode in the laboratory/nerve reference frame ($x,y,z, \theta_x, \theta_y, \theta_z$).

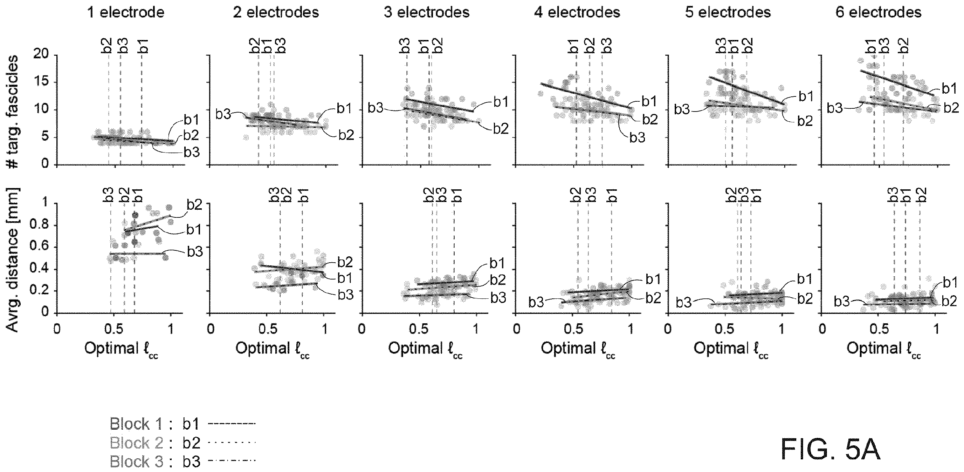

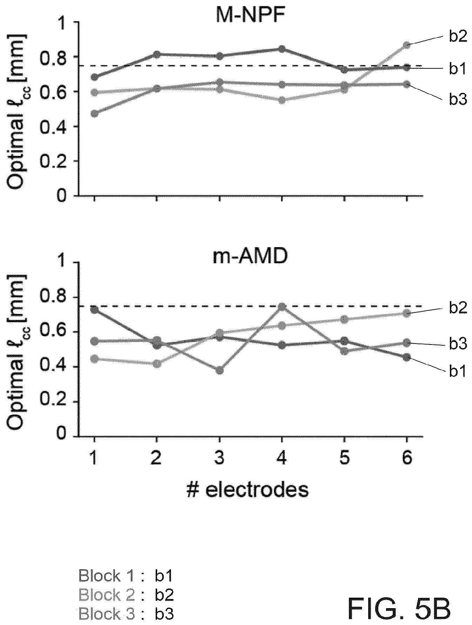

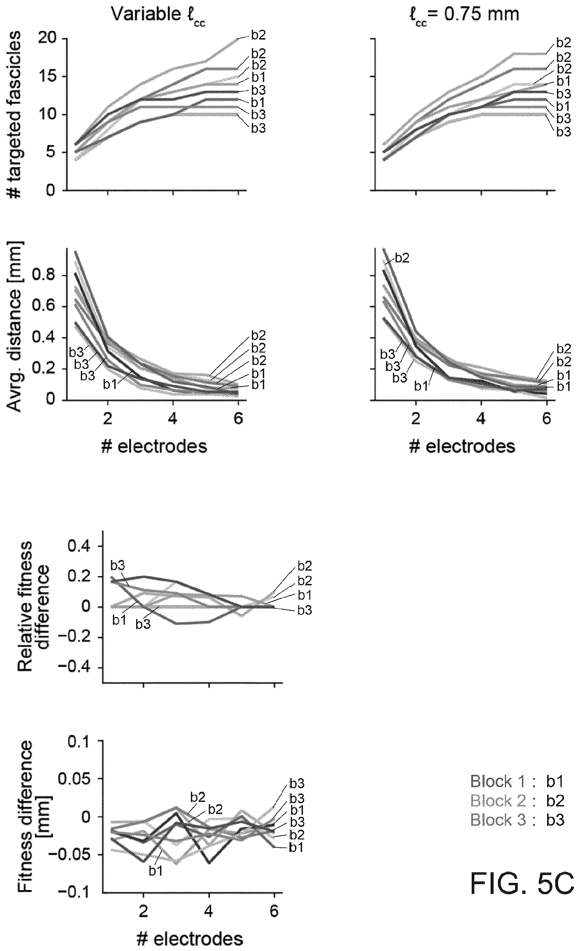

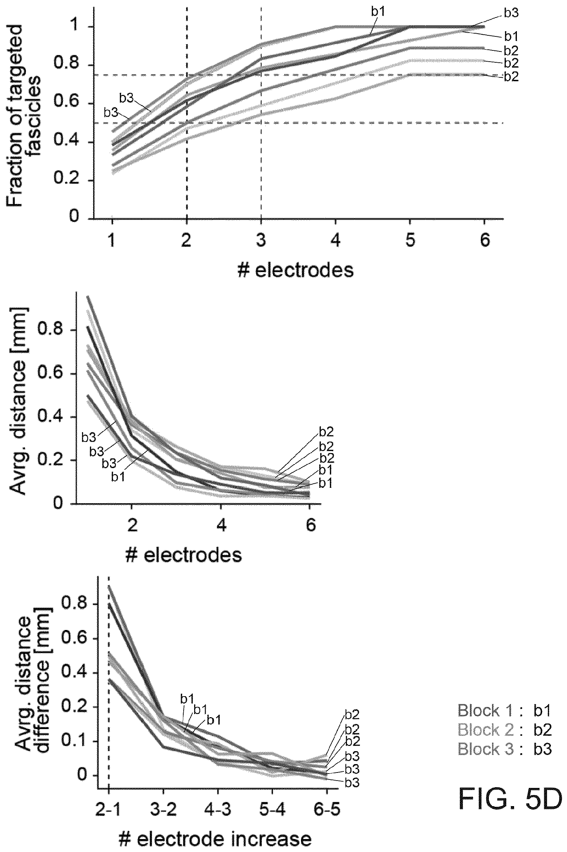

**FIGs. 5A** to **5D** shows different graphs that illustrate the dimensioning and choice of the number of implanted electrodes, with **FIG. 5A** showing a relation between candidate optimal distance between active sites and geometrical fitness for different numbers of electrodes, for «target» (top) and «distance» (bottom) optimization methods, where each point is a candidate optimal implant, the dotted lines correspond to the inter-site distance of the best-fitting geometry for each block **b1, b2, b3,** the dashed lines are obtained by linear regression of the points for each block **b1, b2, b3, FIG. 5B** showing with dashed lines correspond to the optimal inter-site distances for different numbers of electrodes, for «target» (top) and «distance» (bottom) optimization methods, with the black dotted lines corresponding to the TIME intersite-distance currently in use for human applications, **FIG. 5C** showing a fitness of the best-fitting geometries for different number of electrodes, for varying numbers of electrodes and variable or fixed inter-site distance, on the top, the «target» optimization method has been used, while in the bottom we used the «distance» method, the right-most plots show the fitness difference (absolute for «distance», relative for «target») between the best electrodes obtained with variable or with fixed inter-site distance, and **FIG. 5D** showing a choice of the optimal number of implanted electrodes. In the case of «target» optimization method (left), we have traced vertical lines corresponding to two or three implanted electrodes and horizontal lines to targeting 50% or 75% of the fascicles in the sections. In the case of «distance» optimization method (right), we show the fitness increments relative to adding the one implanted electrode at a time and then indicate with a vertical line the step leading to the highest fitness increment.

With respect to the optimization of the implanted electrode design, it is shown how the herein presented geometry optimization routine can be used to provide guidelines to dimension the best electrode for the implant in a given nerve. In **FIG. 5A,** the values of the optimization objective function for the candidate best inter-site distances are shown. Each point is the result of a different algorithm initialization, and we aggregated the results obtained for the different sections in a same block. We show that the candidate best inter-site distances fall into a wide interval (from 0.5 mm to 1 mm approximately), but that lower inter-site distances lead in general to slightly higher performance values. In **FIG. 5B,** the absolute best inter-site distances for different numbers of implanted electrodes are shown. We notice that there is no evident relation between the best inter-site distances and the implanted nerve block or the number of electrodes constituting the implant.

The geometry optimization of the electrodes allows at the same time to evaluate the performance of existing electrode designs with respect to the optimal one. To show this, we ran insertion optimization for implants of electrodes with an exemplary fixed inter-site distance equal to 0.75 mm, corresponding to the electrode design of some clinical trials to restore hand sensation in trans-radial amputees, see reference 13. In **FIG. 5B,** we can see that the optimal inter-site distance is in general lower than the one employed in our existing design. Nonetheless, because in **FIG. 5B** there is a weak dependency of the implant performance upon the employed inter-site distance if the latter belongs to the admissible interval of candidate optimal inter-site distances, we have good reasons to suppose that our fixed design could perform well with respect to the optimized one. In **FIG. 5C,** we thus compared directly the performance of the attainable by the fixed and the optimized design, showing that the loss in performance that derives from using our fixed design is not substantial.

Next, the establishing the best number of implanted electrodes is discussed. The performance curves shown **FIG. 5C** can also be used to establish how many electrodes should be implanted at the different levels along a nerve to obtain the highest performance with the lowest invasiveness. Depending on the objective function employed in the geometrical optimization, different criteria should be formulated. In the case of M-NPF, we set the optimal number of implanted electrodes as the minimal number of electrodes that allows targeting a given proportion of the fascicles. **FIG. 5D** shows that two (2) electrodes allow targeting more than 50% of the fascicles for the two most proximal nerve blocks, but the proportion of pierced fascicles is slightly inferior for the most distal block. If we increase the number of implanted electrodes to three (3), we can target more than 50% of the fascicles for all blocks and more than 75% of the fascicles for the two (2) most proximal blocks. In the case of m-AMD, we can imagine to add one electrode at a time and select the number that grants the maximal increase in performance (elbow method). It can be seen in **FIG. 5D** that two (2) electrodes are the best choice. Generally, since the implant of two (2) electrodes provide adequate performances for both objective functions and because it has been already performed in the human median nerve as shown in references 2,13, whereas three (3) electrodes have never been implanted in a single nerve, we choose to proceed in the following considering the implantation of two (2) electrodes.

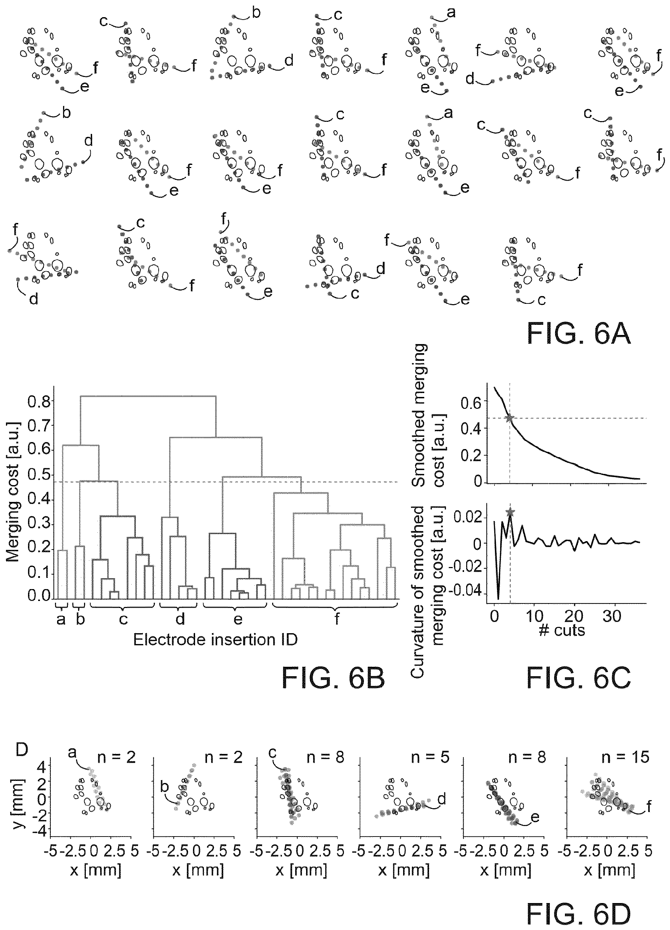

**FIGs. 6A** to **6D** show multiple insertion optimization routines on the same section, with **FIG. 6A** showing optimal implant insertions for a single nerve section. Each subplot corresponds to a random initialization of the optimization algorithm (20 different initializations in total). **FIG. 6B** shows dendrogram for the agglomerative clustering of optimal electrode insertions. The dendrogram is cut at the level of the dashed line, according to what is shown in Panel **FIG. 6C**. FIG. 6C shows the cost of setting different numbers of clusters (n clusters corresponds to performing n - 1 cuts of the dataset) (top) and its curvature (bottom), the number of clusters/cuts were chosen corresponding to the maximum positive curvature of the merging cost, and cut the dendrogram at the corresponding level, and **FIG. 6D** shows different electrode insertion clusters with the corresponding number of electrodes belonging to the clusters.

Next the characterization of best strategies for electrode insertion is discussed. Hereinafter, we show how candidate optimal implant insertions can be analyzed. For brevity, we present the analysis of the candidate optimal implants obtained by M-NPF only, which we chose over m-AMD because intrafascicular stimulation has the potential to recruit selectively muscle groups with very low injected charge. We performed an exemplary twenty (20) independent PSO runs using the first representative nerve section from the most distal nerve block and the resulting implant geometries are shown in **FIG. 6A**. We can see that many electrodes in different implants occupy very similar locations in the nerve section, suggesting that there may be a low number of particularly advantageous single-electrode insertion strategies.

In order to evaluate the clustering of the candidate optimal electrode insertions, we introduced a measure of distance between TIMEs consisting of the average distance of corresponding active sites, and used it to perform clustering and highlight the similarity structures between insertions. **FIG. 6B** shows the dendrogram obtained for the electrodes shown in **FIG. 6A,** and **FIG. 6C** shows how the dendrogram was cut using the elbow method (maximum curvature of the cluster merging cost). The resulting clusters of electrode insertions are shown in **FIG. 6D.** In general, different clusters include different numbers of insertions and display different extents of variability, which can be seen as the robustness of the insertion optimality to variations of the insertion parameters.

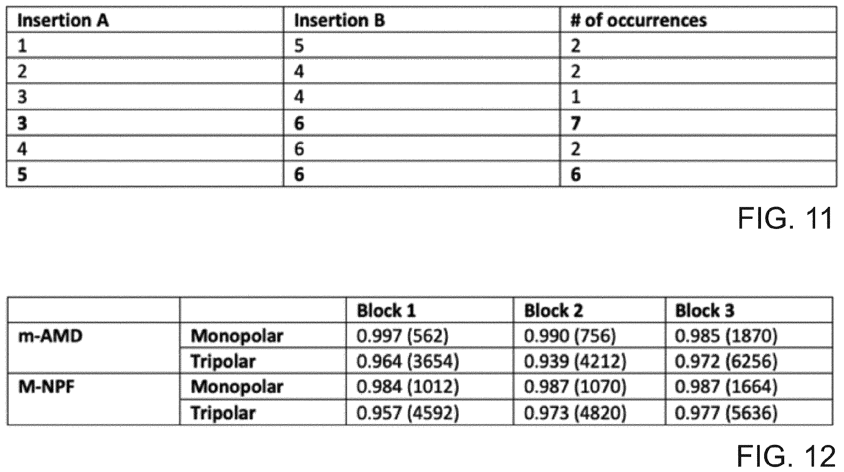

Then, we investigated which couples of electrode insertions occurred most frequently together and show the results in the table shown in **FIG. 11.** Only six (6) possible cluster couplings occur of the thirty (30) possible ones, and the majority of the optimal implants are constructed using electrode insertions from clusters 4 and 6, or 5 and 6. Choosing an insertion strategy from cluster 6 is thus good in general.

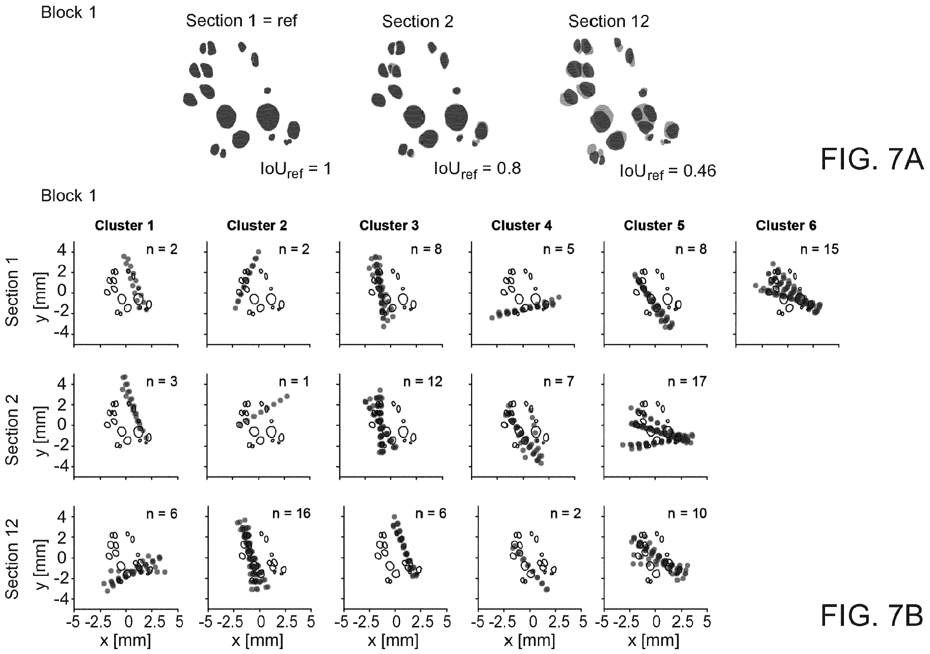

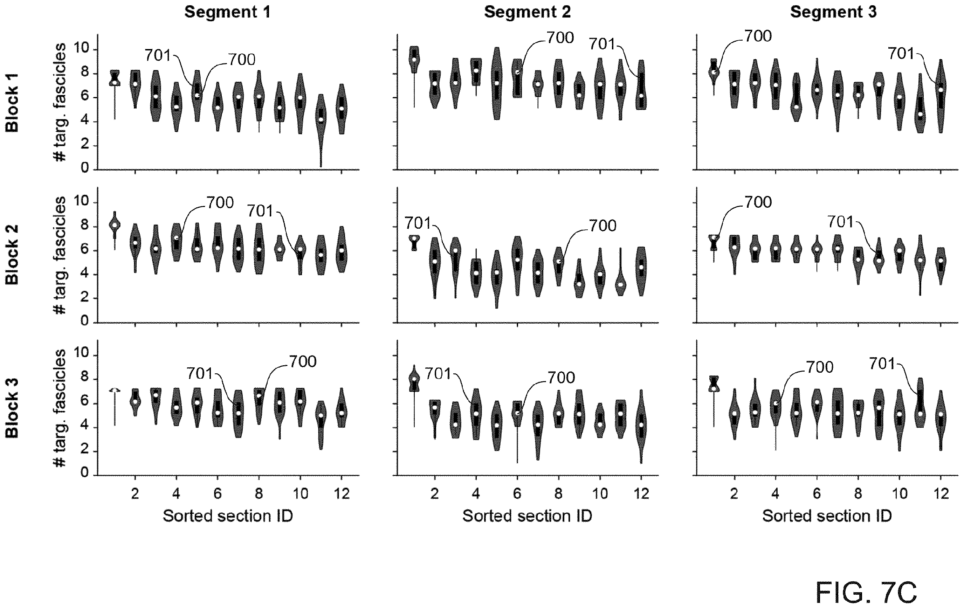

**FIGs. 7A** to **7C** show different representations of the characterization of candidate optimal implant geometries, **FIG. 7A** showing three nerve sections **Section 1, Section 2, Section 12,** from a same nerve segment. The sections are plotted over the reference **Section 1.** The intersection over union (IoU) between each section and the reference section is reported, **FIG. 7B** showing electrode clusters, **cluster 1** to **cluster 6** obtained for the three sections, sections **Section 1, Section 2, Section 12,** where the clusters from each section are presented in no specific order, and the clusters are obtained using the agglomerative clustering method illustrated by **FIGs. 6A** to **6D,** each subplot corresponds to a different cluster, and n indicates the number of electrodes belonging to that cluster (20 optimization runs for each section), and **FIG. 7C** showing a number of fascicles from the representative section of the nerve segment targeted by implants optimized on different nerve sections belonging to the same nerve segment. Each subplot corresponds to a different nerve segment. The violin plot at x-location i corresponds to implants optimized on the i-th section of the segment and evaluated on the reference section of the segment. Each violin plot shows the distribution of the number of pierced fascicles. The white dot **700** represents the median value, and the black rectangle **701** indicates the interquartile range (IQR) of the distribution.

With **FIG. 7A,** we show the clusters of electrode insertions corresponding to different sections sections, **Section 1, Section 2, Section 12,** in the same nerve segment. We can see that in general insertion clusters are robust with respect to small variations in nerve topography. For example, cluster 3 in section 1, cluster 3 in section 2 and cluster 2 in section 12 are very similar. We expanded on this point by assessing the performance of an electrode insertion optimized on a certain nerve section when used in other sections belonging to the same nerve segment. This analysis simulates errors in the pre-operative determination of the nerve fascicular topography. The results are shown in **FIG. 7B.** It can be seen that the decrease in effectiveness of the insertion does not depend strongly upon the dissimilarity between the sections.

For example, we can see that **clusters 3** and **5** are more homogeneous than **cluster 6.** This means that while the insertion of an electrode according to the strategy corresponding to **cluster 3** should be performed very precisely, there is much more freedom in placing an electrode according to the strategy corresponding to **cluster 6.** In fact, even allowing for some uncertainty e.g. in the positioning angle, the electrode insertion will still belong to the cluster.

Next, the training and accuracty of the activation classifier is discussed. A separate multilayer perceptron (MLP) binary classifier was trained for each stimulation setting, including a given nerve and electrode geometry and of the number of active sites that we choose to activate concurrently, for examople monopolar vs. tripolar. To build the training dataset for each classifier, we sampled 10,000 random stimulation protocols as a set of potential stimulation protocols, computed through biophysical modelling which fibers were active, and randomly subsampled the most represented class, for example active or not activem so that we had a balanced dataset. We computed expected classification accuracies performing Monte Carlo cross validation for fifty (50) iterations with a 99%-1% split.

In the table shown in FIG. 12, we report the expected classification accuracies and the number of samples in the balanced dataset corresponding the trained classifiers. We can see that the accuracy values are all above 0.95 except for the single case of block 2 for m-AMD in monopolar stimulation, where the accuracy value was 0.94.

**Results:** with respect to the evaluation of the candidate optimal stimulation protocols, stimulation protocol optimization employs the MLP classifier to predict the pattern of activation corresponding to each candidate stimulation protocol, and progressively selects the protocols leading to high selectivity. In doing so, it could be biased towards regions of the search space where the classifier is not accurate, thus producing stimulation protocols that perform well according to the classifier but do not when applied to the real fiber population. Thus, once the stimulation protocol optimization has identified a number of candidate optimal stimulation protocols, we computed the activation, recruitment and selectivity patterns values obtained via biophysically accurate models. Because we need to evaluate a relatively large pool of candidate, we only model a small part of the total fiber population in the nerve, obtained by random subsampling of each fiber group.

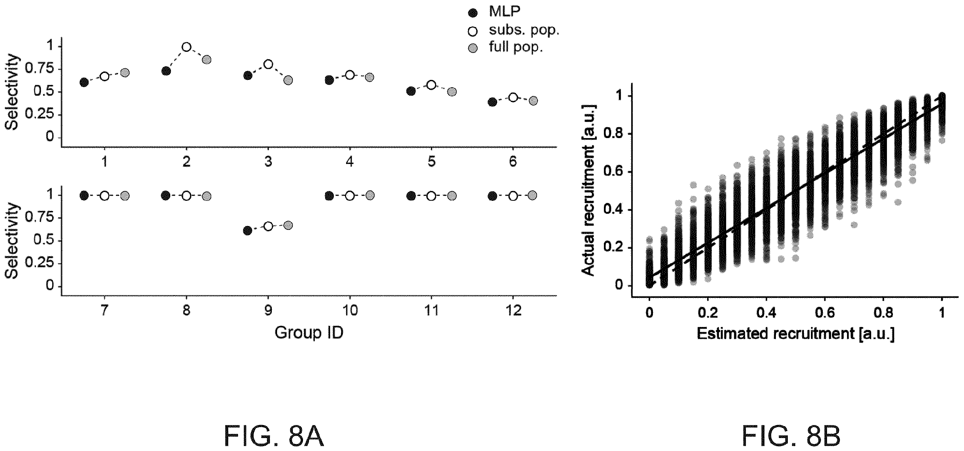

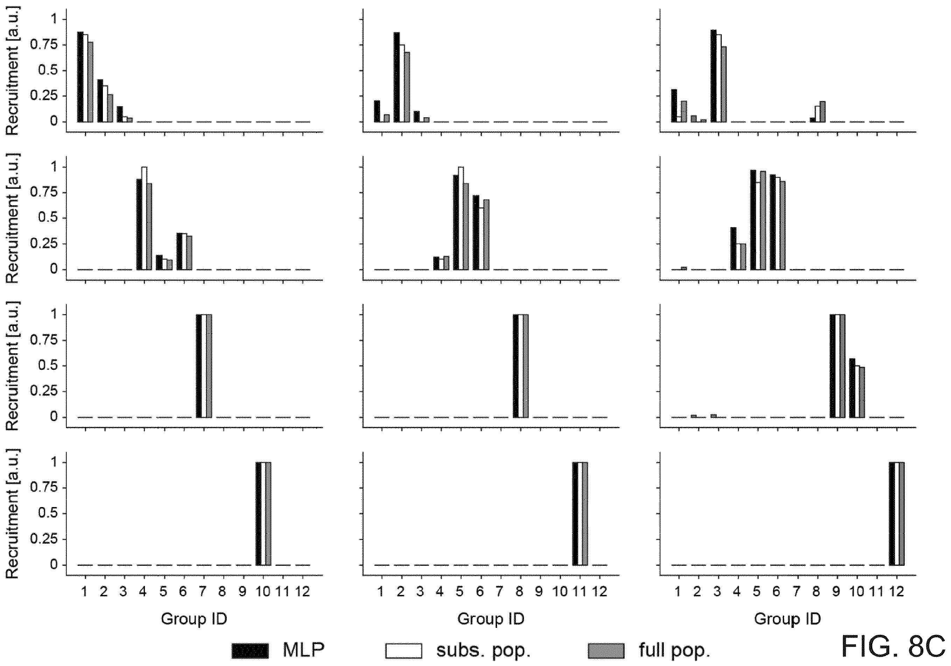

**FIGs. 8A** to **8C** show results of the candidate optimal stimulation protocol evaluation, with **FIG. 8A** showing group-wise selectivity values corresponding to candidate optimal stimulation protocols. For each group we provide (from left to right): selectivity computed using our activation classifier for the five (5) exemplary candidate protocols, selectivity computed using a subsampled fiber population for the five (5) candidate protocols, selectivity computed using the full population for the best protocol determined on the subsampled population, **FIG. 8B** shows the effect of subsampling on recruitment. To determine each point in the scatterplot we activated a random number of fibers in a population and then subsampled it. The y-value of the point is the true recruitment and the x-value is the recruitment estimated from the subsampled population. The solid line is a linear regression on all points, and the dashed line is the quadrant bisector, provided for comparison purposes, and **FIG. 8C** shows recruitment patterns for the best protocols (chosen using subsampling) evaluated with our activation classifier, fiber subsampling, and the full fiber population.

In **FIG. 8A,** we plot the group-wise selectivity values obtained using the three methods. For the MLP classifier and under-sampled estimated selectivities, we plot the best selectivity across five independent re-initializations of the algorithm. We then evaluate the protocol attaining the best selectivity using the full population. We can notice that undersampling the fiber population leads to a systematic overestimation of the selectivity. To better understand this phenomenon, we investigated the effect of under-sampling on recruitment. In **FIG. 8B,** we can see that under-sampling tends to overestimate high recruitment values and to underestimate low recruitment values, which results to a systematic overestimation of the performance of high selectivity stimulation protocols (where the recruitment of the target group is overestimated and the recruitment of the non-target groups is underestimated). Nonetheless, the average error on recruitment is very low for both the under-sampled model and the MLP classifier. In **FIG. 8C,** we display the recruitment patterns estimated using the three methods, corresponding to the highest estimated selectivity for each muscle group.

Futhermore, the optimal stimulation protocols in different conditions are characterized. We performed several in silico experiments to characterize the performance of the optimal stimulation protocols found by our routine in different conditions.

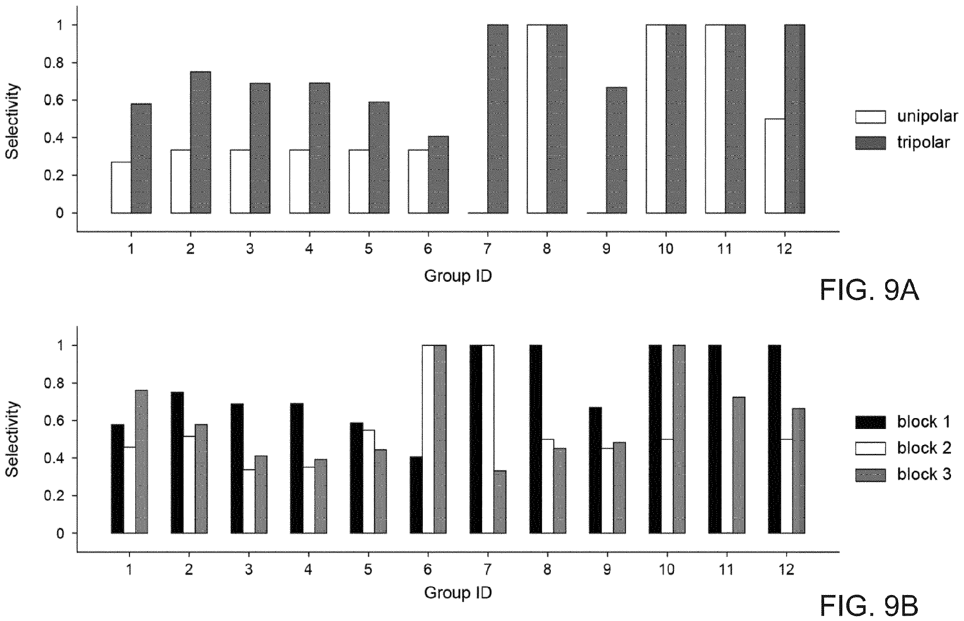

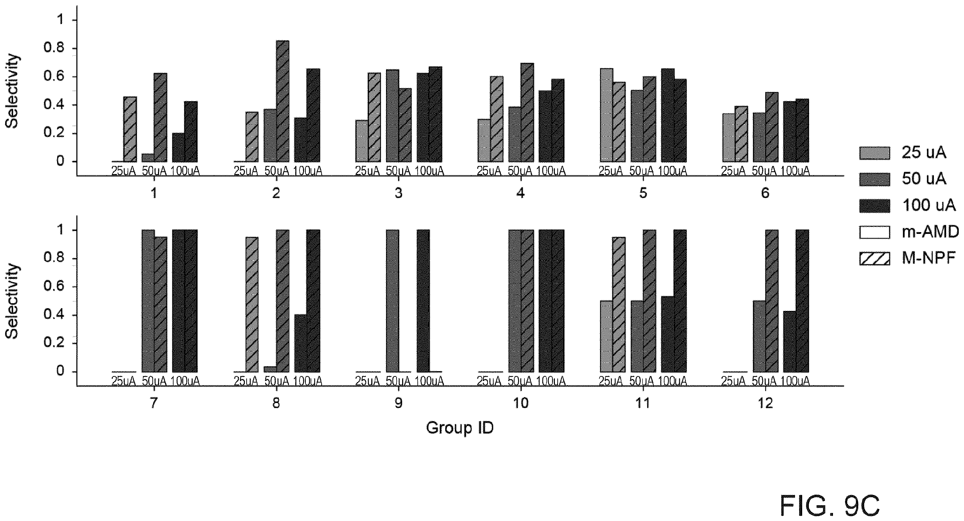

**FIGs. 9A** to **9C** show stimulation protocol optimization in different conditions, with **FIG. 9A** showing a comparison between monopolar and tripolar stimulation, optimization on the reference section from block 1, **FIG. 9B** showing a comparison between tripolar optimal stimulation for the reference section from blocks 1, 2, and 3, and **FIG. 9C** showing a comparison between the best tripolar stimulation for m-AMD and M-NPF optimized implants, when imposing different maximal site-wise current values. All values refer to the reference section from block 1. All selectivity values correspond to the selectivity measure discussed herein.

First, we compared monopolar and tripolar optimal stimulation protocols. Here, with the term "n-polar", we indicate a stimulation protocols were the ground is far away from the stimulation region and n independent currents are applied through n active sites. In general, multipolar stimulation allows to more flexibly shape the electric potential distribution over the nerve section and should thus leads to better selectivity values when fiber groups are spatially segregated. Indeed, in **FIG. 9A,** it can be seen that **unipolar** stimulation is substantially less effective than **tripolar** stimulation. The total selectivity for unipolar stimulation is 0.45 and for tripolar stimulation is 0.78. In the rest of our work, we focus on the optimization of tripolar stimulation.

Second, we compared selectivity in a random representative section from each of the three blocks of the median nerve, corresponding to different distal to proximal levels along the nerve. Studying how the selectivity of our interface changes along the path of the nerve can allow establishing, together with surgical accessibility considerations, the best implantation location. We observe in **FIG. 9B** that the group-wise selectivity has generally the highest values in the most distal block **(block 1),** followed by the most proximal block **(block 3),** while the lowest values correspond to the middle block **(block 2).** This order is maintained in the values of total selectivity: 0.78 for block 1, 0.60 for block 3, and 0.51 for block 2.

Third, we compared the performance of the optimal implants according to **M-NTF** and **m-AMD** fitness functions, in terms of the selectivity of the best stimulation protocols that can be administered from each of them. We performed stimulation protocol optimizations using each of the two implants for the representative section from nerve block 1, constraining to different current magnitude intervals [-50, 50] $\mu$A, [-100, 100] $\mu$A, and [-200, 200] $\mu$A. We tested different current intervals because we expect implants optimized using **M-NPF** to produce selective stimulation protocols for lower current amplitudes than implants optimized using **m-AMD,** because intrafascicular site location is actively encouraged.

The results are shown in **FIG. 9C.** For currents in [-50, 50] μA, we had distance 0.17 and target 0.40, for [-100, 100] (μA we had distance 0.53 and target 0.73, for [-200, 200] (μA we had distance 0.59 and target 0.70. In general, we notice that distance performs worse than target. Distance performs particularly bad for very low current values, while target is more robust, which is coherent with the fact that in target we are generally inside fascicles, and lower current thresholds are observed.

With respect to the effect of suboptimal electrode placement on optimal protocols, the effect of a suboptimal electrode insertion on selectivity was analyzed. We outline three (3) possible scenarios causing suboptimality and compare their impact. First, imperfect electrode placement during surgery, where the actual implant configuration can be obtained from the optimal one for the given nerve section by slightly perturbing the insertion parameters (here $\Delta x$, $\Delta y$, $\Delta\theta_z$). Second, imperfectly predicted fascicular topography. In this scenario the errors are introduced at the level of the topography of the nerve and influence the results of the geometry optimization. The optimal implant will be determined on the base of a fascicular topography different from the actual one, and this will affect the effectiveness of the optimized stimulation protocols. Third, electrodes are randomly inserted in the nerve, with the only constraint that no active site is outside the nerve region. This last scenario gives us an idea of the capabilities of the optimized stimulation protocol alone.

Because the three scenarios produce very different implants, the direct comparison between single group recruitment or selectivity values is not particularly informative. We will thus compare their total selectivity, defined as the average of the maximum group-wise selectivity values [14]. All optimizations were run on the first representative section from block 1, in the hypothesis of tripolar stimulation protocols.

For the first scenario, we considered the optimal implant found for the representative section from block 1, and sampled values for $\Delta x$, $\Delta y$ uniformly in [-0.25, 0.25] mm, and for $\Delta\theta_z$ uniformly in [-15°, 15°] for each of the two implanted electrodes. The x, y, and $\theta_z$ values for the electrodes were changed to the values $(x + \Delta x)$, $(y + \Delta y)$, and $(\theta_z + \Delta\theta_z)$. We optimized the stimulation protocol for the obtained implants and obtained a total selectivity of 0.63.

For the second scenario, we applied to the representative section from block 1 the optimal implants obtained for two different sections of the same nerve block. We chose to employ section 2 and section 12, having an IoU with the reference section of 0.8 and 0.46, respectively. The total selectivity values obtained after optimizing the stimulation protocol were 0.59 and 0.51, respectively.

For the third scenario, we sampled randomly x, y, $\theta_z$ so that no active site was outside a square of side 6 mm centered at the origin. We applied this "random" implant to the representative section from block 1, and optimized the stimulation protocol. The total selectivity value obtained was 0.35.

With respect to the influence of the chosen selectivity definition on stimulation protocol optimization, many different selectivity measures have been defined in the past to assess the quality of stimulation protocols and neuroprosthetic devices. In this section, we show that the choice of the selectivity measure that is used as an objective function in stimulation protocol optimization strongly affects the optimization results, and that our newly defined selectivity measure produces the best results among the state-of-the-art definitions. We consider the three selectivity definitions introduced the references 14, 15, and 16 and we will indicate them as RA, VE, and PO selectivities, respectively.

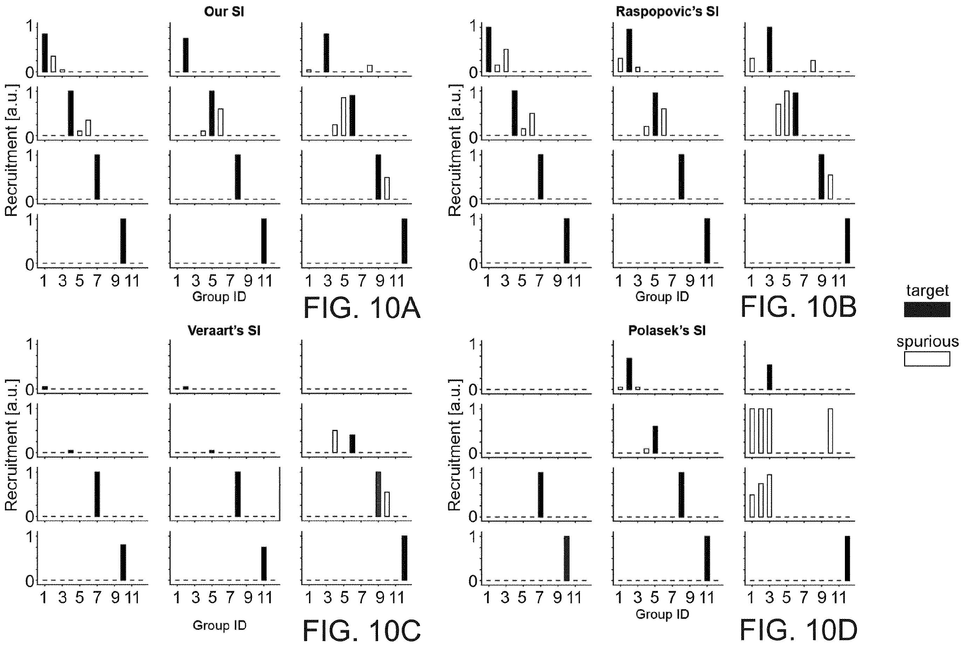

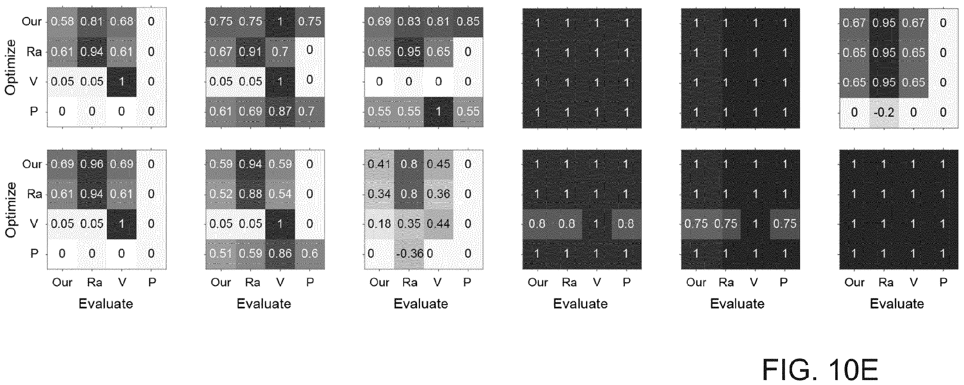

**FIGs. 10A** to **10E** illustrate stimulation protocol optimization for different selectivity measures, in which recruitment patterns for the best tripolar stimulation protocols to stimulate selectively one muscle group at a time maximizing selectivities according to Romeni (see **FIG. 10A),** Raspopovic (see **FIG. 10B),** Veraart (see **FIG. 10C),** Polasek (see **FIG. 10D)** are shown. The i-th plot corresponds to the optimization of the stimulation to optimize group i activation. All recruitment patterns are obtained via fiber population subsampling. **FIG. 10E** depicts selectivity values obtained computing the selectivity according to X corresponding to the protocol optimized maximizing the selectivity according to Y. Each plot corresponds to the optimization of a different muscle group.

In **FIGs. 10A** to **10D,** we can see the recruitment patterns obtained optimizing with respect to the different selectivity measures. In general, recruitment patterns corresponding to RA and our new selectivity definition are mostly equivalent, while the ones corresponding to VE and PO selectivity are substantially worse. Moreover, we see that when optimizing with respect to PO selectivity it can happen to obtain candidate optimal recruitment patterns where non-target muscles are activated while the target one is not. In **FIG. 10E,** we show the value of the different selectivity measures of each stimulation protocol obtained optimizing with respect to each selectivity definition. The best selectivity definition is the one whose maximization produces the better selectivity values for all groups. We compute the performance of a given selectivity definition as the average across groups of the sum of the elements in a given row of the displayed matrices. We obtained 0.77 for our selectivity measure, 0.72 for RA, 0.55 for VE, and 0.57 for PO. We can see that the selectivity measure introduced here gives the better performance, followed closely by RA, while VE and PO selectivities perform substantially worse.

With the herein present method and system, a modular framework to exploit modern optimization techniques is proposed. Specifically, a pipeline is presented for the optimization of the development, planning, implant, and use of intraneural interfaces for neuromodulation. Currently, the main computational bottleneck preventing automatic optimization of stimulation protocols is that every time that the geometry of the implant is modified, the LFM of the electrical problem has to be recomputed, which requires a lot of time and limits in practice the number of objective function evaluations. We avoid this bottleneck by separating the optimization of the implant geometry and of the stimulation protocol. Namely, the implant geometry is optimized using only geometrical considerations; the optimal implant geometry is used to build the LFM of the problem; and is then used to train the surrogate model of fiber activation that is employed in stimulation protocol optimization.

According to an aspect of the present invention, the herein presented method and system can be generalized using different optimization methods and objective functions for the two optimization steps. Different objective functions can be more appropriate in different situations, for example, M-NPF is an objective function specifically devised for the optimization of intraneural implant geometries, but m-AMD can be employed for extraneural implants as well. Equally, it is possible to think about different objective functions for stimulation protocol optimization, for example, trying to match specific recruitment patterns, which could lead to stimulation protocols to produce graded force patterns, while here we were only interested in maximizing group selectivity and thus aimed at maximal recruitment for the target groups.

The choice of employing a MLP or more generally ML methods to predict fiber activation is also not structural to our framework. Any surrogate model of fiber activation can be employed, including the existing model-based methods based on the activating function formalism. Nonetheless, we believe that ML will allow building more and more complex surrogate models in the future, that can capture the full nonlinear dynamics displayed by electrically stimulated nerve fibers. For example, it is straightforward to generalize our MLP to predict fiber firing-rates, while it is impossible to extend the activating function formalism to obtain such results.

We briefly stated in the *results* paragraph herein above that the joint use of surrogate models for the prediction of fiber activation and of black-box optimization to compute the optimal recruitment or selectivity patterns could produce optimal "adversarial examples". It is in fact theoretically possible for the optimizer to push optimization towards regions of the search space where performances are particularly high because of the inaccurate behavior of the surrogate model. Here, we want to underline that this phenomenon is structural to the use of surrogate models in computing the objective function of an optimizer, and it is not linked specifically to our use of ML methods. Nonetheless, it is possible that in general theoretically grounded surrogate models may have a more regular behavior, and that they are less subject to the abovementioned phenomenon. With the herein presented method and system, it was shown that adversarial examples occur rarely with our MLP activation classifier, but further analyses should be performed to quantify in a more systematic way these effects. Finally, we remark that the possible presence of these adversarial examples does not make our performances less reliable, but leads simply to the underestimation of the performances attainable if the true optima were found. The results presented in the present work are thus pessimistic estimates of what could have been found with more powerful ML surrogate models and a higher number of optimization re-initializations.

According to another aspect of the present invention, with the herein presented method and system, it is possible to establish and evaluate invasive implant geometry. Specifically, the method can be used to produce the optimal electrode inter-site distance for TIMEs. It is interesting to notice that the optimal inter-site distance does not decrease systematically when increasing the number of implanted electrodes. This is reasonable for the m-NPF approach, as there is no incentive for lower numbers of electrodes to span wider areas of the nerve. Nonetheless, we would have expected generally higher inter-site distances to be selected by m-AMD when a lower number of stimulating sites was available, because intuitively it could be argued that it is more relevant in those cases to distribute the available sites to "cover" maximally the nerve section. The fact that this is not the case suggests how these geometry optimization processes can benefit from quantitative approaches and should not be performed manually on the base of common sense. Common sense can provide acceptable solutions, but it can fail in the presence of geometrical/physical trade-offs.

If specific electrode geometries are already available and they differ substantially from the optimal ones, the potential loss in performance due to employing suboptimal geometries should be evaluated. In fact, even if the candidate optimal implants can differ systematically from the existing ones, it is still possible that fitness of the existing geometry is not substantially lower than the optimal one. In this case, available electrodes may thus be preferred out of convenience.

The number of electrodes to be implanted can be established by performing separate optimizations with different numbers of electrodes and by comparing the best fitness values obtained, as shown in **FIGs. 5C** and **5D**. While it is mathematically possible to optimize directly the number of electrodes including it into the optimization variables for the implant, evolution would favor implants containing a higher number of electrodes, as they contain a higher number of stimulating sites and will have a higher likelihood of providing by chance better values of the objective function. The same can be said regarding the optimization of the number of stimulating site per electrode, which we have not shown for brevity. Alternatively, a regularization term could be added to the objective function, to penalize implants with higher numbers of electrodes or stimulating sites, which, in turn, requires fixing the regularization constant. Moreover, because the best implant with n electrodes is at least as good as the best implant with n-1 electrodes, but is more invasive, the best number of electrodes cannot be determined by looking at objective function values only.

Also, it can be determined what the best implant site should be. The problem of where the implant should be performed along the longitudinal axis of the nerve can be studied performing optimization on nerve sections obtained from different locations. Some preliminary considerations can be made by observing the fitness values of optimal implant geometries at different levels along the nerve. In **FIGs. 5C** and **5D,** we can see that the number of fascicles targeted by the optimal implants grows proceeding proximally, even if the proportion of targeted fascicles grows proceeding distally. This makes it difficult to choose the best implant location based solely on geometrical considerations. Specifically, if we decide to implant the most proximal block of the nerve, we are able to target a very high percentage of fascicles, but because the absolute number of fascicles is lower than for distal sections, those targeted fascicles will contain on average a higher number of muscle groups, whose selective activation can be challenging. On the contrary, in sections located more distally, the proportion of targeted fascicles is lower, so, on average, we expect to have active sites close to a lower number of muscle groups. The targeted group, nonetheless, are most likely to be the only group in the targeted fascicle, which allows selective stimulation.

This trade-off can be solved by performing stimulation protocol optimization. In **FIG. 9B,** it was found that the best implantation site was the most distal section, followed by the most proximal, and finally by the central section. A similar analysis has been presented in Badi, Wurth and colleagues with the reference 17. There, the optimal insertion location of a single TIME in the median nerve of a macaque monkey was determined by computing the total selectivity corresponding to the best monopolar stimulation protocols. Three different levels along the nerve proximally to the elbow joint have been analyzed, obtaining that the middle block led to the best selectivity. The higher selectivity attained in our study for the distal section may be justified by noting that we implanted twice the number of active sites, in such a way to maximize the number of targeted fascicles, while in reference 17 the insertion was manually set. This may lead to a substantially better coverage of the many nerve fascicles and to a higher selectivity. The improvement in performance for the most proximal section, instead, can be justified by the fact that we employed multipolar stimulation protocols, which allow coping with multi-group fascicles thanks to electric field steering, see for example **FIG. 6A,** and also the additional discussion in the section related to the paragraph *multipolar stimulation that leads to substantially higher selectivity* herein below.

Finally, we notice that the site of implantation can also determine the optimal implant geometry. In **FIG. 5D,** while two electrodes target a satisfactory portion of the fascicles in **block 2** and **block 3**, for **block 1** three electrodes would have likely performed better, at the cost of a higher invasiveness, whose opportunity should be carefully evaluated.

Moreover, according to another aspect of the present invention, the herein presented method and system allows estimating the best electrode insertion strategy and its robustness. For example, the optimization routines outputs insertion strategies that are local optima for the selected fitness function. Nevertheless, analyzing the different output local optima (which we have called "candidate" local optima) can help us understand what the landscape of optimal implants is. Interestingly, the candidate optimal implants output by our routine display high similarity. Single electrode insertion strategies can be clustered in a low number of recurring optimal insertions, and the intra-cluster variability suggests us how sensitive that optimal insertion strategy is to geometrical variations. When we analyzed couples of electrode insertions, we saw that the number of represented implants is actually even lower.

We compared the optimal implants obtained from different variations of our representative nerve sections, and noticed that the candidate optimal electrode insertions for similar nerve sections are in general very similar. This nonetheless did not prevent their fitness when used on the reference section (so, a different section with respect to the one they were computed from), the geometrical fitness diminished immediately and stabilized for higher variations of the section, maintaining throughout the different sections a very high variability. From these results only, it would seem that our optimal geometries are extremely sensitive to errors in the determination of the nerve fascicular topography. Nonetheless, we should notice that the target fitness function is per se particularly sensitive for differences in the nerve topography, because it decreases in steps as active sites fall outside of fascicles. It is possible to imagine very subtle variations between the estimated and the true topographies, which make so that our active sites are very close but outside the "targeted" fascicle boundaries, and thus count as non-targeted fascicles. To quantify the true robustness of our implants, we added stimulation protocol optimization and evaluate the loss in performance, in terms of decrease in total selectivity, given by implant suboptimality. Indeed, if the large decrease in geometrical fitness is due to the sensitivity of our fitness function and not to an actual huge worsening of the electrodes placement, the decrease in total selectivity should be much less marked.

Moreover, not every selectivity definition of the state of the art can be used in black-box optimization. We can observe how some selectivity measures have evident drawbacks that prevent their effective use in optimization routines. Specifically, VE selectivity measure does not take into account the absolute value of group recruitments, which has the effect to favor very small activations of single groups, giving them unitary selectivity values (see e.g. groups 1, 2, 4, and 5). PO measure is too strict and assigns zero selectivity to coactivations. This leads to evaluating in the same way many suboptimal but not equivalent patterns, leading to nonsensical candidate optima (see e.g. groups 6 and 9). RA measure, and the one proposed in this study allow obtaining very similar recruitment patterns and are both adequate to be used in black-box optimization. Stimulation protocol optimization based on RA measure outputs in general very high selectivity values when there are many muscle groups, even in presence of strong coactivation of a few groups and thus the «functional» quality of the underlying recruitment pattern is not immediately interpretable. Our selectivity measure is much more conservative in this respect, but it is dimensionally incorrect and thus more difficult to interpret. In general, the maximization of our selectivity measure produces the recruitment patterns that are evaluated as the best by all other selectivity measures on average. RA selectivity is inferior but comparable.

**Multipolar stimulation that leads to substantially higher selectivity** : also, we have shown that multipolar stimulation leads to substantially higher selectivity. For example, we found that multipolar stimulation attains in general higher degrees of selectivity than unipolar stimulation. This was expected, as the former allows more freedom to shape the generated electric potential field through the superposition of the actions of different active sites. Moreover, in our routine, every unipolar stimulation protocol is a valid tripolar stimulation protocol, where one active site applies a non-null current and the other active sites apply a null current. This alone grants that if our algorithm were capable to determine the global optimum, and the global optimum were a unipolar stimulation protocol, it would be found by our algorithm. Thus, we would have that the selectivity for tripolar stimulation is necessarily higher or equal than the selectivity for monopolar stimulation. Nonetheless, because of the imperfections in our optimization routine, this result was not obvious. Indeed, the search space is larger in multipolar stimulation, and the optimization landscape more complex, which could have led to worse performance. The fact that our result matches the theoretical expectations is a sanity check of our optimization routine. Moreover, while multipolar stimulation cannot perform worse than monopolar stimulation in a theoretical setting, the extent of the improvement could have been negligible. We see that this is not the case. Further investigations in multisite stimulation protocols should be carried on, to study the impact of the number of independently controllable stimulating sites and the maximal attainable selectivity. Such analyses may help to understand if substantial technological advancements are needed to solve the problems related to increasing the number of independently controllable stimulating sites.

For each setting, we computed an exemplary number of five (5) candidate-optimal stimulation protocols through independent initializations of our optimization routine, which estimates fiber activation using the binary classifiers described above. Then, we computed the selectivity values (according to our definition, unless stated otherwise) corresponding to the activation patterns found applying the candidate-optimal stimulation protocols to a population of biophysically accurate fibers subsampled of a factor of ten (10), with twenty (20) fibers for each group, for a total of 240 fibers.

In general, monopolar stimulation datasets lead to smaller datasets, most likely because higher currents are required to activate fibers using a single electrode (the maximum injected charge is three times lower). Still, the attained accuracy values were higher than in the case of tripolar stimulation. This suggests that the prediction of activation under monopolar stimulation is simpler than under tripolar stimulation, which is reasonable because the dimension of the input space is smaller.

With respect to other characterizations of the herein presented method and system, and future developments, it is assumed that the fascicular topography at different levels of a nerve can be established before surgery, with a precision sufficient to run the method for the implant geometry localization routine. While the non-invasive determination of nerve fascicular topography has been shown using magnetic resonance 18 and ultra-high frequency ultrasound 19 imaging, its precision in the reliable reconstruction of fascicle geometries has never been quantified. The herein presented method and system shows that such imaging techniques should be actively developed and characterized, as they can allow pre-surgical planning resulting in the substantial increase in the selectivity of the implanted devices.

When optimizing the stimulation protocols, we assumed to know exactly the functional topography of the implanted nerve section. While intuitively the approximate location of different functional groups can be determined by single-site stimulation using stimulating sites in different locations in the implanted electrodes and employing triangulation, similarly to what is done during recording in the references 20 and 21, to the best of our knowledge, no methods have been developed yet. We have shown that the availability of a precise functional topography of the nerve allows attaining extremely good selectivity patterns, thus encouraging the investigation for such function localization methods.

The sensitivity of our results with respect to different functional topographies should be investigated. Three-dimensional (3D) reconstruction algorithms for the fascicular topography of a nerve, and fiber tracing algorithms should be used to compare the stimulation at different levels of the same nerve in such a way that respects functional group migrations along the nerve.

Activation classifier approaches should be explored further using hyper-parameter optimization and possibly active learning protocols that allow building directly balanced/informative datasets in terms of the underlying stimulation patterns, which would allow a much lower number of biophysically-detailed simulations needed.

The presented approach is currently directed to applications where fiber functional groups can be univocally defined, and thus recruitments and selectivities can be estimated. This corresponds essentially to applications such as movement restoration, where each functional group corresponds to a different innervated muscle, and bioelectronic medicine, where each functional group corresponds to a bodily function or an innervated organ. In the case of bioelectronics medicine, an additional constraint is given by the fact that the nerve functional topography should be determined without modulating vital organ functions in a blind way, and can be identified using for example the discriminative beamforming method presented in reference 21, which employs recordings of neural spontaneous activity. Nonetheless, sensory restoration poses a very different challenge, in that the different single fibers account for sensations that span a continuum, and thus, while our whole abstract framework can be applied to this problem, an adequate objective function for stimulation protocol optimization should be determined and validated.

In sum, a method and sytem is presented that allows to optimize intraneural neuroprostheses for movement restoration and bioelectronic medicine. We were able to optimize in silico the number of implanted electrodes, their geometries, their insertions in the nerve transverse section, the level along the nerve where the implant should be implanted, and the stimulation protocol that targets a given functional group with maximal selectivity. We provide motivation to develop reliable and precise methods for the non-invasive or minimally invasive determination of nerve fascicular and functional topographies, which can allow the use of HMs to optimize neural interface. The method and system paves the way for true personalized optimization pipelines thus moving the role of modelling from qualitative assessment of different stimulation settings to being a fundamental tool in the development, implantation, and use of neuroprosthetic devices.

Next, additional details and implementations of the steps of the method are provided.

With respect to the implant geometry optimization, and the geometrical parametrization of electrode geometry and insertion, we model TIMEs using three (3) geometrical parameters, and six (6) insertion parameters. The geometrical parameters are the number of active sites $n_{as}$ per face, the face-to-face distance $\ell_{ff}$, the center to center distance $\ell_{cc}$ between consecutive active sites on a same face of the TIME. The insertion parameters describe the location $(x, y, z)$ and orientation $(\theta_x, \theta_y, \theta_z)$ of the electrode reference frame with respect to the nerve or laboratory reference frame.

With the herein present method and system, we assumed to optimize a transverse implant procedure ($\theta_x = \theta_y = 0, z = 0$), and constrained the geometry of the electrodes to have $\ell_{ff} = 0.01$ mm, and $n_{as} = 8$. These values correspond to the ones employed in [13]. The value of $\ell_{ff}$ cannot be easily modified as it corresponds to the depth of the folded polyimide layer constituting the electrode body; the value of $n_{as}$ can be modified, and its choice should be operated in the same way in which we chose the number of electrodes to be implanted. For the sake of brevity, we did not perform this last analyses.

In the first in silico experiment, we optimized both the insertion $(x, y, \theta_z)$ for each implanted electrode and the inter-site distance $\ell_{cc}$ common to all the electrodes in the implant. Next, we set $\ell_{cc} = 0.75$ mm [13] and optimized only the insertion parameters for each electrode. The optimization variables $x$ and $y$ were constrained in the interval [-5, 5] mm, $\theta_z$ was contrained in the interval [0, $2\pi$], and $\ell_{cc}$ in [0, 1] mm

With respect to the implant geometry optimization and objective funtions, the objective function M-NPF corresponds to the number of fascicles containing at least one implant stimulating site. We chose this function because, since a poorly conducting perineurium sheath surrounds fascicles, it is very easy to target selectively a single fascicle when being inside of it. Because of the relatively high number of muscle groups with respect to the number of fascicles in the human median nerve, we have good reason to believe that most muscle groups will be segregated in different fascicles, and that stimulating intrafascicularly will increase our functional selectivity (see also [17]).

The objective function m-AMD is computed as the average of the distance between each fascicle and the closest stimulating site in the implant. This function allows having at least one active site very close to each nerve fascicle, thus being able to stimulate with relatively low intensity thresholds all fascicles. This objective function is particularly useful in the case in which it is not possible to target all fascicles, because while M-NPF assigns a zero contribution to all not targeted fascicles, in this case we can have a meaningful indication about the distance between sites and fascicles and minimize it.

With respect to the stimulation protocol optimization, and the stimulation protocol parametrization, the current amplitude of each active site was constrained in the range [-250, 250] µA for tripolar stimulation and [-250, 0] µA for monopolar stimulation, unless stated otherwise. We chose a symmetric interval of amplitudes in the case of tripolar stimulation in order to allow current steering; when performing monopolar stimulation, negative current (cathodic) stimulation was chosen because it displays lower threshold values for fiber activation.

In order to optimize n-site stimulation without the need to decide beforehand which n sites among all available sites will be used for stimulation, we evolve a set of weights together with the stimulation amplitudes. Each weight refers to a single site, weights are constrained to vary in [0, 1], and for each candidate solution, the actual stimulation protocol is given by the amplitudes of the n stimulating sites with the n highest values for the weights.

With respect to the stimulation protocol optimization objective function, The objective function for stimulation protocol optimization is the maximization of a selectivity measure that we define as

$$Sel_{g,s} = \frac{\eta_{g,s}^2}{\sum_{g'} \eta_{g',s}}$$

where $n_{g,s}$ denotes the value of the recruitment for group $g$ and stimulation protocol $s$. See below for more details.

In the last in silico experiment, we also perform stimulation protocol optimization maximizing other selectivity measures from the literature. See below for more details.

**PSO parameters employed:** for the optimizations, we used the PSO algorithm implemented by the MATLAB function "particleswarm" . For the geometry optimizations, we used a swarm size of fifty (50) and performed 150 iterations, for the stimulation protocol optimization, we used a swarm size of twenty-five (25) and performed fifty (50) iterations. The swarm size and number of iterations were chosen so that the fitness values generally converged before the last iteration. In the case of stimulation protocol optimization, swarm size and number of iteration are lower because estimating the activation of the whole fiber population is in general a slower process than the purely geometrical evaluation of the implant geometry fitness.

With respect to median nerve fascicular and functional topographies, concerning the fascicular topography, we define a nerve fascicular topography as the set of polylines defining fascicle contours in a nerve section. Fascicular topographies have been obtained from the manual segmentation of the nerve sections presented in 12. We considered the sections belonging to blocks 10 (here denoted as block 1), 11 (here block 2), and 12 (here block 3), which correspond to locations above the elbow, where fiber groups targeting all the muscles innervated by the median nerve are still present. While it was known that each block consisted of three separate nerve segments, the remaining section labeling was deemed unreliable after visual inspection of the sections gross features. Thus, we performed registration and clustering to recover nerve segments with relatively stable topography. For each section in a block, we sampled uniform point clouds in the fascicle regions and determined the optimal affine transformation between any couple of masks using the iterative closest point (ICP) algorithm implemented in the MATLAB function pcregistericp. We evaluated the quality of the registration using the intersection over union (IoU) of the registered fascicular polyshapes. We performed agglomerative clustering using as a distance between sections d = (1 - IoU), and cut the dendrogram to obtain three clusters for each block, ideally corresponding to the initial nerve segments. For each cluster/segment, we used the values of the IoU to determine a representative mask/section (the one having lowest average IoU distance from the others in the cluster) and ordered the other sections in the segment by decreasing IoU with the reference one.

With respect to the simulated functional topography, we indicate the assignment of each fiber in the nerve section with a functional group with the expression functional topography. We defined an exemplary twelve (12) muscle groups, corresponding to the twelve (12) muscles controlled by the median nerve. Such muscles were taken from reference 12, and were opponens pollicis (OP), abductor pollicis brevis (APB), flexor pollicis brevis (FPB), first lumbrical (1L), second lumbrical (2L), flexor digitorum superficialis (FDS), pronator quadratus (PQ), flexor digitorum profundus (FDP), flexor pollicis longus (FPL), palmaris longus (PL), flexor carpi radiali (FCR), pronator teres (PT). Muscles coming first in this list are the muscles that branch out of the nerve more distally, and that thus occupy preferentially more central locations in the nerve section. Some groups were subdivided in three major branches containing OP, APB, and FPB; 1L, and 2L; PQ, FDP, and FPL. The other groups did not belong to any major branch and were considered independent when establishing the functional topography. We placed 25,000 fibers uniformly in the nerve fascicles. We divided fascicles into:

- very small-sized fascicles (surface area < 0.05 mm$^2$), which were not assigned to any muscle group;
- small-sized fascicles (surface area between 0.05 mm$^2$ and 0.5 mm$^2$), which were assigned a maximum of one (1) muscle group;
- medium-sized fascicles (surface area between 0.5 mm$^2$ and 1 mm$^2$), which were assigned a maximum of three (3) muscle groups;
- large-sized fascicles (surface area larger than 1 mm$^2$), which were assigned a maximum of five (5) muscle groups.

The number of free groups is equal to

$$n_{free} = n_{small} + 3 \cdot n_{medium} + 5 \cdot n_{large}$$

We ordered fascicles with respect to their distance to the centroid of the fascicular topography and eliminated from the list of the free groups the

$$n_{prohibited} = \frac{n_{free} - 12}{2}$$

fascicles nearest to the section centroid. This corresponds to the fact that we expect in general that sensory fibers going to the hand will branch out more distally than any other fiber in the nerve section and will thus occupy the most central locations distally.

We then assigned muscles to free groups so that muscles constituting a major branch stay in a same fascicle if at the moment of their assignment there is still a fascicle with the right number of free groups. Otherwise, one random muscle at a time is removed from the branch and a fascicle able to contain the largest sub-branch is searched, and so on.

For each fascicle, fibers are assigned to the muscle groups in the fascicle one at a time. We select the group to which we will assign a fiber by sampling a group identifier with a probability proportional to the number of fibers left to assign to a group. Then, we sample the fiber to assign to the group by sampling a fiber with probability proportional to the value of the gaussian density function assigned to the given muscle to each fiber.

With respect to the fiber node location, we consider forty (40) internodes for each fiber. Fiber diameters have been sampled uniformly in [12, 20] μA. Because fiber internode length in the MRG model depends upon the fiber diameter, so does the total fiber length, and this needs to be taken into account when establishing the length of the modelled segment of nerve in the FEM. Fibers nodes are subdivided into four regions: the lower boundary, the FEM, the propagation, and the upper boundary regions. We apply the extracellular potential determined via FEM at the nodes belonging to the FEM regions, and we record the membrane potential from the last Ranvier node in the propagation region (towards the upper boundary region). The size of the FEM region is set in millimeters, and it corresponds to the region where the extracellular potential is substantially different from zero. Thus, because we are fixing the total number of internodes of the fibers, we obtain different numbers of nodes in the FEM region for different fibers. The number of nodes in the propagation region is fixed and set to five, so that the signal passes across the same number of "regeneration steps" at the active Ranvier nodes for each fiber. The propagation region assures that the action potentials observed at the recording node are self-sustained and will not vanish travelling a long length along the nerve, finally getting to the target muscles. The remaining fiber nodes are subdivided into the lower and upper boundary regions. Those regions are necessary to avoid edge effects linked to the fact that we are simulated a very short fiber length on the recorded membrane potential.

With respect to the computation of the lead field matrix (LFM), the LFM of the electrical problem is computed via finite element modelling as described in reference 22. Specifically, the geometrical model of the nerve is built extruding the polylinear fascicular topography of a nerve section; point current sources are distributed in the nerve according to the geometry of an implant determined via implant geometry optimization; a saline bath surrounds the nerve and its external surface is grounded. One FEM solution is computed for each stimulating site in the implant, with a single stimulating site injecting a 1 μA current and all other sites switched off. The resulting potential map is interpolated using COMSOL interpolation mechanism to obtain the extracellular electric potential corresponding to fiber nodes. We computed the extrusion length for the nerve geometry as the length of the shortest fiber in our population. In this way, there will be nodes in the fiber FEM region that are actually outside the COMSOL geometry. We set the extracellular potential applied to these nodes to zero. Even for very large nerve extrusions lengths, the precision imposed to COMSOL computed potential introduces very slight discontinuities in the field between the last node in the COMSOL geometry and the first one outside of it. These discontinuities may be amplified when we compute the extracellular potential pattern corresponding to different stimulation protocols, and thus perturb the action potential generation and propagation. We smoothen the LFM by applying a bump window function to the non-zero extracellular potential applied to the fiber.

With respect to the activation, recruitment, and selectivity, specifically the activation, a nerve fiber subject to a given stimulation protocol is considered active if at least a spike propagates to a sufficiently far away distance from the stimulation site. The presence of a spike is identified by checking for the presence of peaks higher that 10 mV with prominence 60 mV in the membrane potential time-course. In order to establish that we are recording the membrane potential time-course at a distance far enough from the stimulation site, we visually check that the membrane potential at the recording site is not strongly affected by the stimulation pulse. If this is the case, we can assume that the impulse will successfully propagate to its target.

With respect to the activation, recruitment, and selectivity, specifically the recruitment, the recruitment value $\eta_{g,s}$ corresponding to a fiber group $g$ and a stimulation protocol $s$ is computed as the fraction of active fibers in the group, or

$$\eta_{g,s} = n_{g,s}^{\mathrm{act}}/n_g$$

With respect to the activation, recruitment, and selectivity, specifically the selectivity, many definitions of stimulation selectivity have been provided in the literature. Here, we study four possible definitions of stimulation selectivity, defined by Raspopovic and colleagues 14, Veraart and colleagues 15, Polasek and colleagues 16, and ourselves.

RA selectivity value corresponding to a stimulation protocol s and a fiber group $g$ is given by

$$Sel_{g,s}^{RA} = \eta_{g,s} - \frac{1}{N_g - 1} \sum_{g' \neq g} \eta_{g',s} = \eta_{g,s} - \mathrm{mean}[\eta_{g',s}]$$

This selectivity measure varies in the interval [-1, 1], It has a value of -1 when the target group has null recruitment and all other groups have unitary recruitment, 0 when the target group has a recruitment equal to the average recruitment from all other groups, and 1 when the target group is fully recruited while all other groups have null recruitment. When the value is positive, we are recruiting the target group more than we are recruiting other groups on average; and when it is negative, we are recruiting the target group less than the other groups on average. Nonetheless, the penalization of co-activation of a few groups is lower and lower the more we increase the number of groups, because the contribution of the few co-activated groups is lowered by averaging them with very low recruitment values. For example, consider a recruitment pattern where out of eleven muscles we have the target muscle and an antagonistic muscle both fully recruited and all the other groups not recruited. RA selectivity value is 0.9 but the recruitment pattern is not functional and it should intuitively correspond to very low selectivity values.

VE selectivity value corresponding to a stimulation protocol s and a fiber group $g$ is given by

$$Sel_{g,s}^{VE} = \eta_{g,s} \bigg/ \sum_{g'} \eta_{g',s}$$

This selectivity measure varies in the interval [0, 1]. It is 0 when the recruitment of the target group is null, and 1 when the target group is the only recruited group. While this measure penalized harshly co-activation, in practical situations it tends to favor very low target group recruitments. Using low amplitudes, in fact, the stimulation is more localized and can lead to very low recruitments of a single group, which lead to unitary selectivity.

PO selectivity value corresponding to a stimulation protocol s and a fiber group $g$ is given by the recruitment of the group if no other group is recruited more than a given threshold value (0.1 in Polasek et al 16, 0.2 in Brill et al 23),

$$Sel_{g,s}^{PO} = \begin{cases} \eta_{g,s}, & if\ \eta_{g',s} \leq \eta_{thr}, for\ all\ g' \neq g \\ 0, & otherwise \end{cases}$$

This measure varies in the interval [0, 1], and is extremely strict with respect of co-activation. For this reason, we expected it not to be suitable for stimulation protocol black-box optimization, as it does not provide informative evaluation of suboptimal stimulation protocols, which would drive the optimization towards optimal regions. For example, target group full recruitment, another group with recruitment 0.21 and all others will null recruitment gives selectivity 0, as a pattern where the target group is not recruited and all other groups are maximally recruited. Thus, the algorithm can only hope to already be in an optimal region of the optimization space, which happens extremely rarely, leading to a very high variability of the optimal protocol selectivity for different search initializations.

We introduce a new selectivity measure, which solves the principal issue of VE selectivity (the fact that it maximal for very low recruitment values of the target group recruitment if it is the most recruited one), at the cost of maybe less interpretable selectivity values. We define the selectivity values corresponding to a stimulation protocol s and a fiber group $g$ as

$$Sel_{g,s} = \eta_{g,s}^2 \bigg/ \sum_{g'} \eta_{g',s}$$

In this case, the recruitment pattern $\eta_s = [0.1, 0, 0, 0, ...]$ which gives a unitary VE selectivity, gives selectivity 0.1.

Next, we will describe how an encoder-decoder neural network can be trained to act as a machine learning-based surrogate model of the FEM. We built 5 nerve morphologies in which each morphology contained a random number of fascicles between 3 and 7, whose radii were chosen randomly between 0.2 mm and 0.5 mm, for a nerve radius of 2 mm. We located 100 potential stimulating sites, modelled as point currents, in each of the nerve morphologies, in the plane z = 0. Then, we computed the LFM corresponding to a regular 64 × 64 grid in such plane. Finally, we built a datasets containing 100,000 samples corresponding to different stimulation protocols employing 1 to 5 concurrently active stimulation sites and normalized random stimulation currents whose magnitudes ranged between -1 and 1, on three of the five generated nerve morphologies. We thus trained the 2D UNet and computed the predicted electric potential fields corresponding to a set of stimulation protocols on the unseen nerve morphologies to test the obtained surrogate models of the FEM.

The inputs to the 2D UNet were tensors constituted by two bidimensional arrays. The first bidimensional array represents the nerve morphology and contains a 1 for each location inside a nerve fascicle and a 0 for each location outside a nerve fascicle. The second bidimensional array represents the stimulating electrode locations and the applied currents: the value at each location is 0 if there is no stimulating electrode implanted in that location or if the electrode implanted in that location is not applying a stimulation current, and corresponds to the applied current intensity (positive or negative) otherwise. The outputs from the 2D UNet were bidimensional arrays containing the electric potential at the different locations inside the nerve section.

The network was trained using the MSE loss, the Adam optimizer with learning rate 10^-2, for 5 epochs.

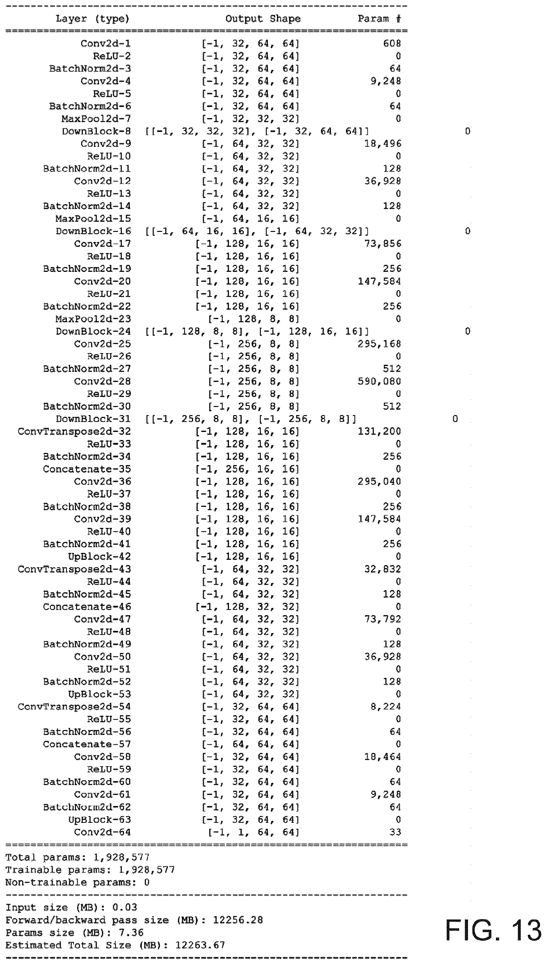

In **FIG. 13** we show a summary of the employed UNet for 2D outputs.

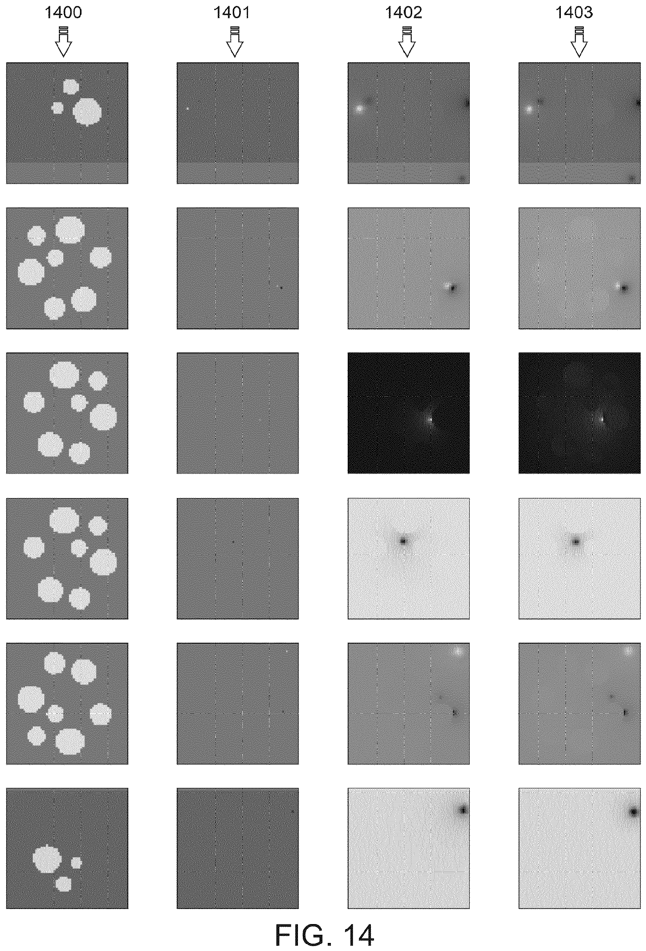

In **FIG. 14** we show examples of the nerve morphologies **1400** (fascicular topography), of the stimulation patterns **1401** (electrode locations and stimulation currents), of the true map **1402** (potential map obtained through FME) of the electric potential in the plane containing the stimulating electrodes, and of the predicted electric potential **1403** (potential obtained through 2D-UNet). We highlight the very high fidelity of the predicted electric potential to the actual one. **FIG. 14** shows the performance of a trained surrogate model of the FEM on a subset of the test set. In the different columns are shown, from left to right, a nerve morphology encoded in a binary array whose entries are 1 for the fascicles and 0 for the extrafascicular medium; a set of stimulating electrodes and a stimulation protocol, encoded as a real-valued array, with non-zero entries corresponding to the active electrodes, with associated values corresponding to the applied current amplitudes normalized in the range (-1, 1); a potential map obtained through FEM; and a corresponding potential map determined through the UNet surrogate model. Both potential maps are encoded as real-valued arrays.

Next, we will describe how to employ a MLP for the regression of neural units firing rates in the case of periodic stimulation, and CNNs for the classification of neural units activation in the case of aperiodic stimulation. Both the above problems have been solved here in the case where all neural elements are homogeneous, namely, they have the same identity (all neural elements are optic nerve fibers) and topology (all biophysical models are unbranched cable with the same number of Ranvier nodes). When the stimulation applied to the target structure by a set of stimulating electrodes consists of a set of periodic trains of rectangular pulses, or of sinusoidal waves with a frequency common to all stimulating electrodes but different current magnitudes, we can uniquely associate to the set of site locations with respect to the target neural element (x, y, z for each electrode), of site current magnitudes (one value for each electrode), and the stimulation frequency (one value common to all electrodes) to a corresponding firing rate. Thus, they are used respectively as the input and outputs to train a MLP. Equivalently, we could employ a CNN that accepts as input a bidimensional array containing the values of the extracellular electric potential at each node of the neural elements, at each time instant. Such spatio-temporal image of the stimulation potential is then used to predict either the value of the activation or of the firing rate of the neural element. When computing the spatio-temporal electric potential array, there is no mathematical reason to constrain the stimulation to be periodic, and we thus trained as an example a binary activation classifier that employs a CNN architecture.

We applied the firing rate MLP regressor and the activation CNN classifier to the problem of predicting the behavior of optic nerve fibers, whose biophysical model has been taken from 24 and 25.

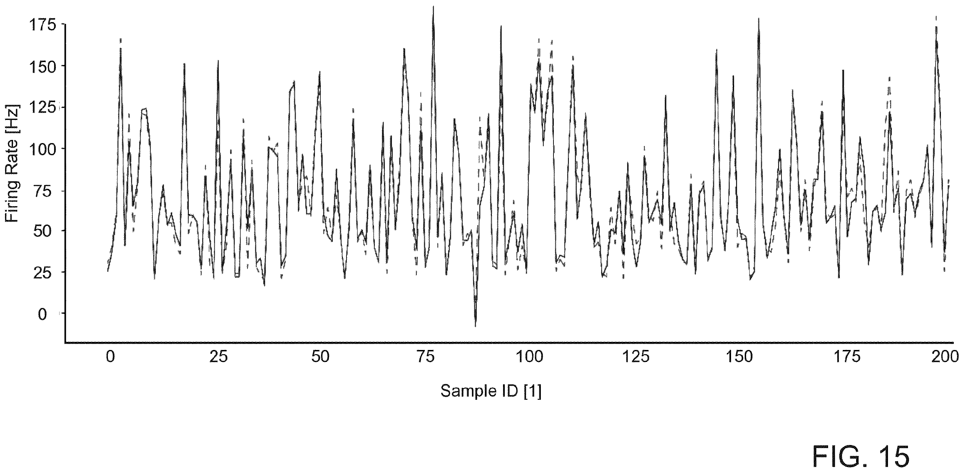

In **FIG. 15,** we show the actual and predicted firing rates for a subset of the employed test set in the firing rate regression using a MLP. **FIG. 15** shows the performance of a trained MLP surrogate model for the prediction of nerve fiber firing rates under periodic stimulation protocols on a subset of the test set. The solid line indicates the firing rates obtained through a biophysically accurate model of nerve fiber, while the dashed line indificates the firing rates obtained through the surrogate model.

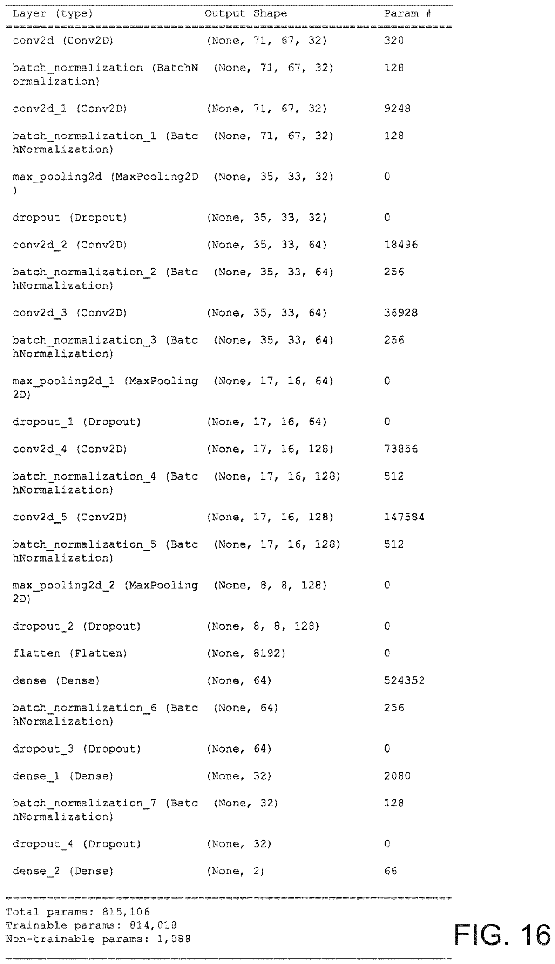

In **FIG. 16,** we show a summary of the CNN model employed to perform the activation classification under aperiodic electrical stimulation.

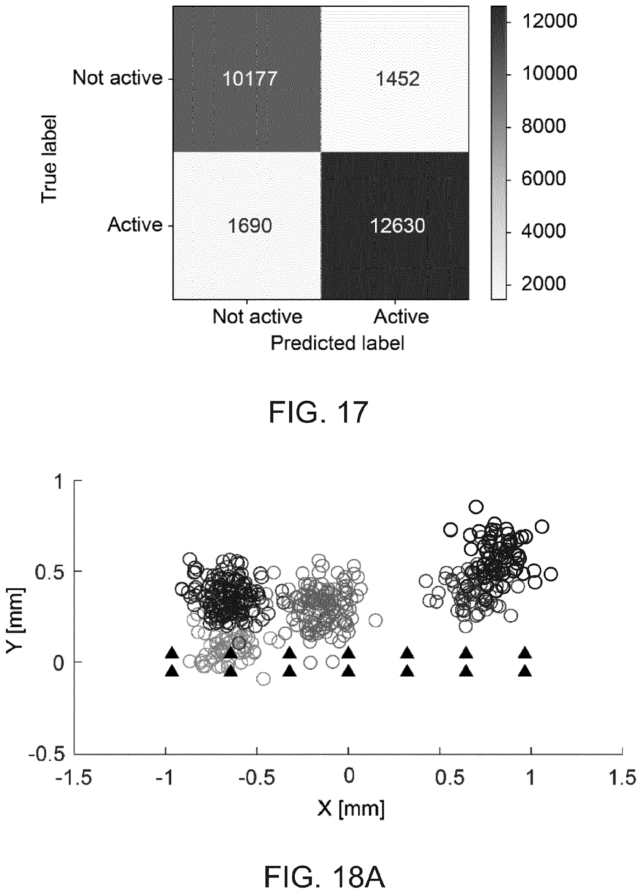

In **FIG. 17,** we show the confusion matrix for the activation classification on the employed test set.

Next, we describe how it is possible to perform functional topography estimation through monopolar stimulations of a motor nerve. Suppose that a peripheral nerve innervates several muscles, then we are interested to determine the point in the nerve section which has the highest likelihood of being at the center of the cluster of fibers targeting a specific muscle. Experimentally, the recruitment curve for the muscle has to be determined. Monopolar stimulation protocols with increasing amplitudes are applied to the nerve from each one of the implant electrodes, and the value of the recruitment of the muscle is estimated as the electromyographic (EMG) signal power normalized by the maximum power elicitable through electrical stimulation. From these recruitment curves we can establish a value $I_i^{half}$ for each electrode, which corresponds to the current amplitude injected by the given electrode which is required to obtain a recruitment of 0.5. A set of discriminative coefficients $D_i$ are computed as the reciprocals of $I_i^{half}$ for each electrode. A computational model of the nerve stimulation is built and the LFM $L(r)$ is computed. A calibration phase is carried on, where we locate a very high number of clusters of fibers $C_j$ in the nerve section. Then, we compute the set of discriminative coefficients $D_i^j$. We cap the $L_i(r)$ at a cap value $\theta_k$ for different $k$, and obtain

$$[\hat{L}_i^k(r)] = \begin{cases} [L_i(r)], & if\ [L_i(r)] < \theta_k \\ \theta_k, & otherwise \end{cases}$$

then we compute the localization map as

$$\ell^{jk}[r] = \sum_i D_i^j \cdot \hat{L}_i^k(r)$$

and the hypothesized location of the cluster is given by

$$r^{jk} = \underset{r'}{\operatorname{argmax}}\ \ell^{jk}(r').$$

Then, we choose the cap

$$\theta_k^j = \underset{k'}{\operatorname{argmin}}\ \varepsilon_{k'}^j,$$

where

$$\varepsilon_k^j = d(r^{jk}, \tilde{r}^j)$$

is the localization error, or the distance between the true and hypothesized location of the cluster. We thus associate for each cluster the value $\underset{i}{\max} D_i^j$ with the optimal capping value $\theta_k^j$, and perform linear interpolation to produce a straight line associating each possible value of the maximum discriminability index in the electrode array to the optimal capping value for the LFM. Finally we compute the value $\underset{i}{\max} D_i$ for the cluster to be localized, and obtain its center location by applying the equations above using the optimal capping obtained from the linear regressor.

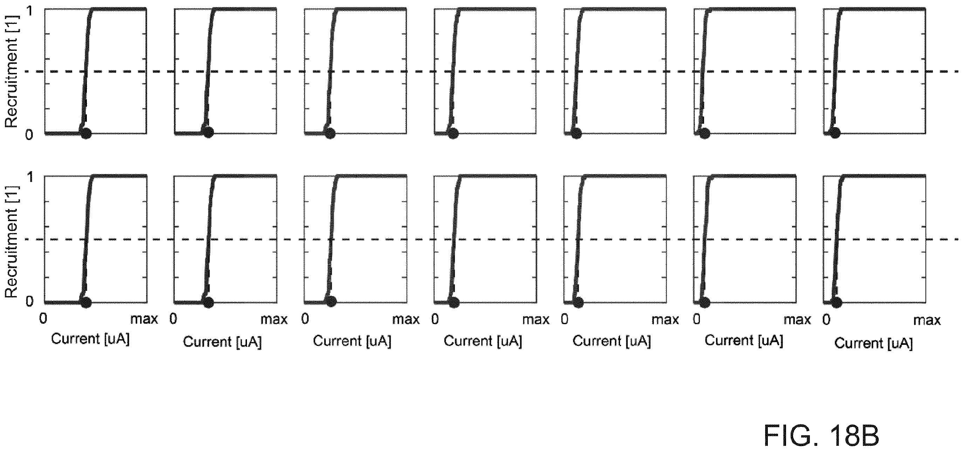

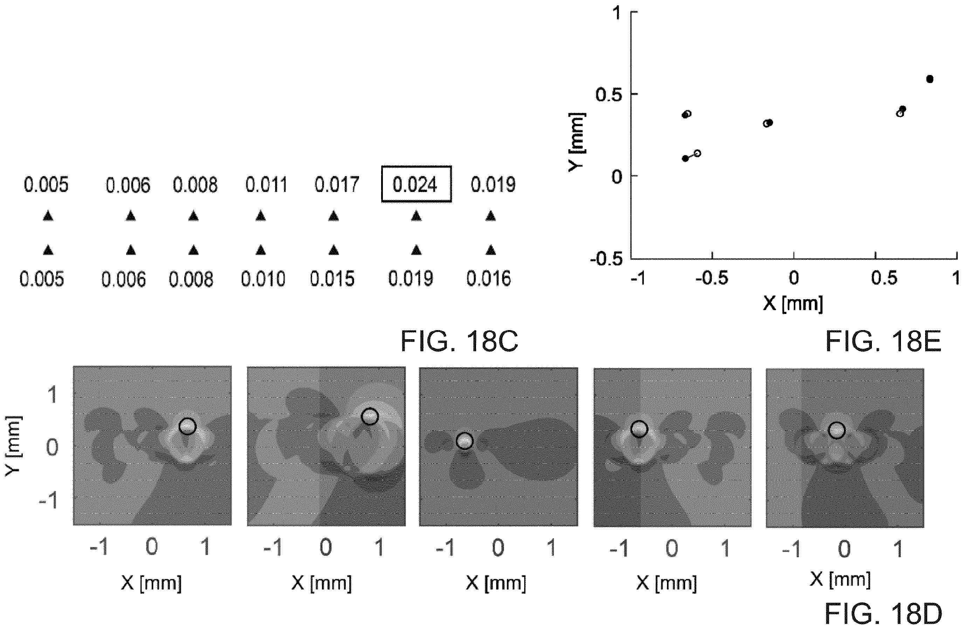

**FIGs. 18A** to **18E** show the different phases of motor fiber cluster localization in the section of a peripheral nerve using stimulation. **FIG. 18A** shows a simulated functional topography of a peripheral nerve, with each circle marker indicating a nerve fiber, each different shading indicating different cluster of fibers, and each triangle marker indicating a stimulating electrode. **FIG. 18B** shows the recruitment curves for one cluster of fiber and each stimulating electrode. Additionally, the level of recruitment 0.5 has been highlighted with a dotted line, and the corresponding I half current has been indicated with a filled circle marker. **FIG. 18C** shows the discriminative indices 1/I_half associated to each stimulating site; the highest value is highlighted and should be used to fix the lead field matrix cap. **FIG. 18D** shows the localization maps for each of the five groups in our example: the maximum of the localization maps corresponds to the recovered locations of each cluster, here highlighted with a black circle. **FIG. 18E** shows the true (filled circle) and guessed (empty circle) locations of the clusters of fibers.

In **FIGs. 18A-E** we can see the results of a simulation of the localization method using monopolar stimulation. In **FIG. 18A,** we show the simulation set-up: five fiber groups and 14 stimulating electrodes are dispersed in a homogeneous, isotropic, infinite medium with the electrical properties of the neural tissue. In **FIG. 18B,** we show the recruitment curves obtained by computing the fraction of active fibers when each stimulating electrode injects a current from 0 to a given maximum value. Here, we have established which fibers are active by thresholding the electric potential map in correspondence to the fiber locations, coherently with the mirror function formalism presented in 26. In **FIG. 18C,** we show the values of the reciprocals of the currents that produce recruitment 0.5, or the discriminability index values. We highlight the highest value, as it will establish the value of the cap to be applied to the lead field matrix (procedure not shown here). In **FIG. 18D,** we show the localization maps for each group, highlighting the location of their maxima, which correspond to the guessed group center location. In **FIG. 18E,** we show the localization error, or the distance between the true and guessed group center locations. We can see how the localization errors are extremely low with respect to the typical distances in the problem.

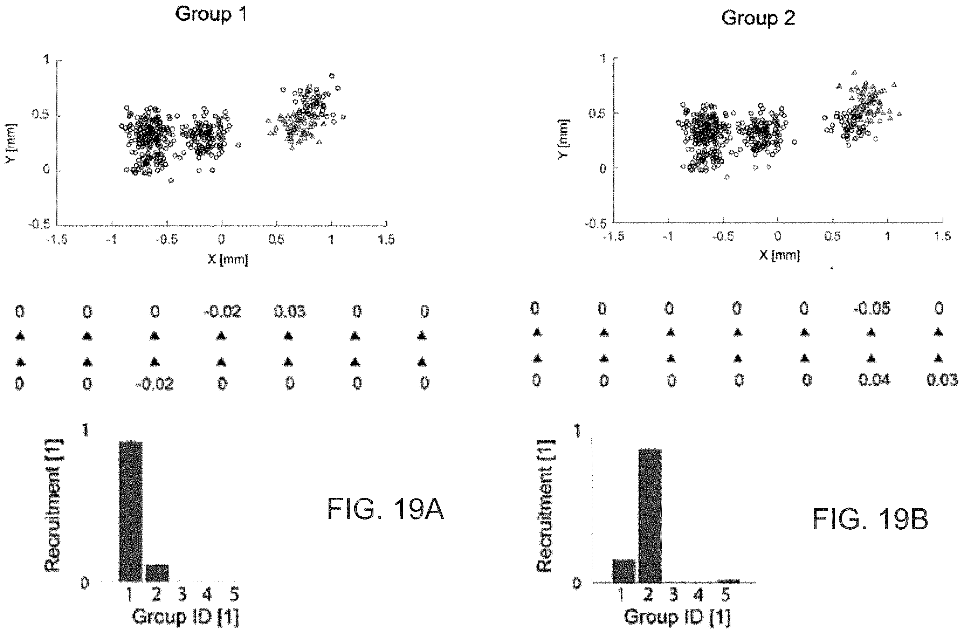

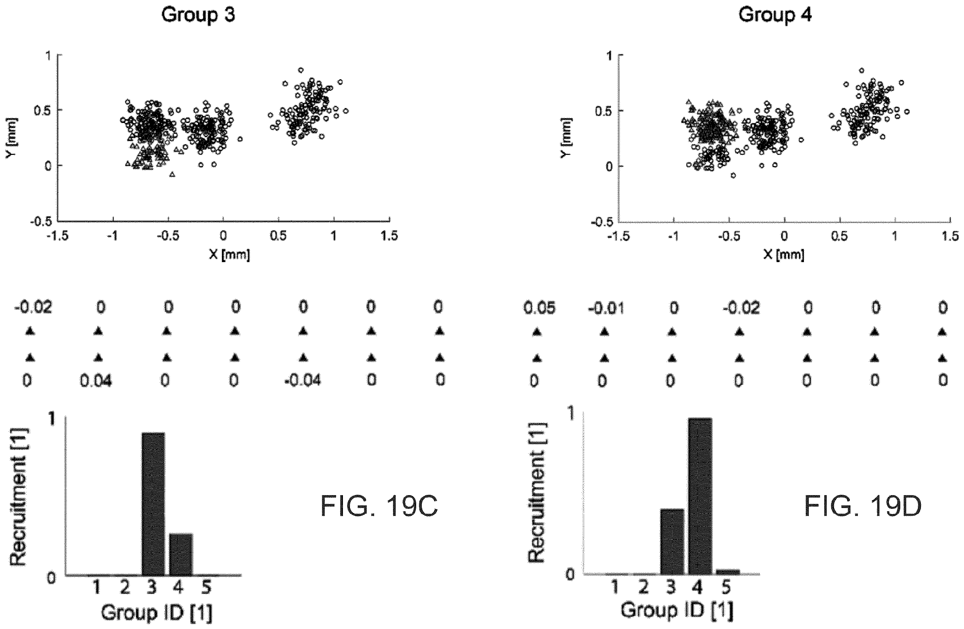

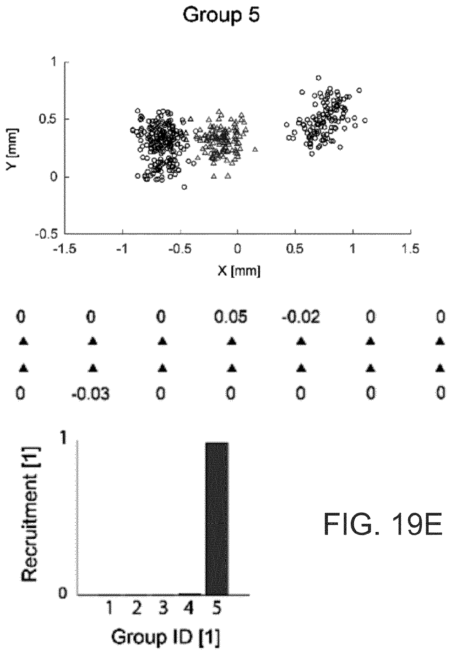

Next, we describe how it is possible to exploit the information obtained through localization to optimize the stimulation protocol applied to the target neural structure. We show the results of an in-silico experiment which uses the same set-up shown in **FIGs. 18A-E.** The localization of the groups are given by the center locations output by the localization routine. We thus sample a set of reasonable fiber locations around these center locations with a given spatial dispersion (fiber locations are sampled according to a bidimensional gaussian distribution). We will use these fiber locations to optimize our stimulation protocols, as during the optimization phase the true fiber locations are unknown. The optimization is analogous to the one presented in **FIGs. 4** to **12,** but is operated now on fictitious fiber populations. Here we show that the selectivity produced by the optimal stimulation protocols optimized on the basis of the available imperfect information about the distribution of the neural element functional topography is still satisfactory. We use a PSO to optimize tripolar stimulation protocols so that the selectivity defined as the ratio between the squared value of the target group recruitment and the sum of all group recruitments be maximized. **FIGS. 19A-E** show the results of the stimulation optimization to maximize the stimulation selectivity of each fiber group in **FIGs. 18A-E,** using the localization shown in **FIGs. 18A-E** to establish the location of the groups. For each group, we show the fibers activated by the stimulation protocol (light marker: active fibers, dark marker: inactive fibers, triangular marker: target fibers, circular marker: off-target fibers), the optimal stimulation protocols (in arbitrary units, tripolar stimulation), and the recruitment array (value of the proportion of recruited fibers for each group). In **FIGs. 19A-E,** we can see the results in terms of activation and recruitment patterns corresponding to the optimal protocol on the true population (while the protocol was optimized on the guessed population), and the corresponding optimal stimulation protocol. We can see from the recruitment patterns that the selectivities are very high in that the target groups show substantially higher recruitment values for every choice of the target group.

NOMENCLATURE

In the herein present specification, the following terms have been used.

Nerve morphology: It encompasses some or all the geometrical characteristics of a nerve section. Specifically, it can be seen as the characterization of the nerve fascicular and functional topographies along the portion of the nerve that is modelled. Here, we will assume that no variation of fascicular of functional topographies happens along the nerve, or that the nerve morphology for a given portion of a nerve is completely determined given a single transverse section.

Nerve fascicular topography: It consists of the location and extent of the fascicles present in a nerve section. Here we define it as the set of the polylines defining fascicles in a nerve section, as we use the polylinear fascicle approximation.

Nerve functional topography: It consists of the association between the locations of the fibers in a nerve section and the function they are related to. While it is in general difficult to define "functional groups" in an unambiguous way, here, we deal with groups of fibers innervating different muscles as different functional groups.

Implant: In this work, we will call implant a set of electrodes implanted on a same nerve portion.

Implant geometry: It is a specification of the geometries and insertions of the electrodes composing an implant.

Electrode geometry: It is the geometry of each electrode in itself. Here, it is determined by the values of the electrode geometrical parameters, for example but not limited to (1) number of active sites per electrode face, (2) center-to-center distance between two neighboring electrode sites, and (3) distance between the two faces of the electrode.

Electrode insertion: It is the geometry of each electrode with respect to the nerve. If we imagine the geometry of an electrode to be given in an electrode reference frame, insertion consists of the geometrical transformation that maps each electrode reference frame to the laboratory reference frame, centered in the centroid of the nerve fascicular topography, with z axis along the axis of the nerve.

Lead field matrix of the volume conduction problem: It is a matrix containing a number of rows equal to the number of fiber nodes and a number of columns equal to the number of stimulating sites in the electrode. Entry $(i,j)$ correspond to the potential at fiber node $i$ when electrode site $j$ applies a unitary current stimulation to the nerve and all the other electrode sites are switched off. It can be multiplied by the simulation protocol to obtain the corresponding potential at all fiber nodes.

Stimulation protocol: Here, it is simply an array of steady-state current values, one for each stimulating site in the implant. If only one stimulating site is on, the stimulation protocol is called unipolar, otherwise it is called multipolar.

Recruitment pattern: It is an array containing the recruitment values for each fiber group in a nerve section, corresponding to a single stimulation protocol.

Fiber nodes: The points along a fiber where the extracellular electric potential is applied. They correspond to the centers of the sections in which the fibers are divided. Here, they correspond to the centers of the nodes of Ranvier, MYSAs, FLUTs, STINs composing the fibers (see Methods).

While the invention has been disclosed with reference to certain preferred embodiments, numerous modifications, alterations, and changes to the described embodiments, and equivalents thereof, are possible without departing from the sphere and scope of the invention. Accordingly, it is intended that the invention not be limited to the described embodiments, and be given the broadest reasonable interpretation in accordance with the language of the appended claims.

REFERENCES

1. Slavin, K. V. Peripheral nerve stimulation for neuropathic pain. 5, 7 (2008).

2. Raspopovic, S. *et al.* Restoring Natural Sensory Feedback in Real-Time Bidirectional Hand Prostheses. *Sci. Transl. Med.* **6**, (2014).

3. Valle, G., Petrini, F. M., Mijovic, P., Mijovic, B. & Raspopovic, S. A Computer-Brain Interface that Restores Lost Extremities' Touch and Movement Sensations. in Brain-Computer Interface Research (eds. Guger, C., Allison, B. Z. & Gunduz, A.) 65-73 (Springer International Publishing, 2021). doi:10.1007/978-3-030-79287-9_7.

4. Rowald, A. et al. Activity-dependent spinal cord neuromodulation rapidly restores trunk and leg motor functions after complete paralysis. Nat Med 28, 260-271 (2022).

5. Benabid, A. L. Deep brain stimulation for Parkinson's disease. Current Opinion in Neurobiology 13, 696-706 (2003).

6. Raspopovic, S., Capogrosso, M., Badia, J., Navarro, X. & Micera, S. Experimental Validation of a Hybrid Computational Model for Selective Stimulation Using Transverse Intrafascicular Multichannel Electrodes. IEEE Transactions on Neural Systems and Rehabilitation Engineering 20, 395-404 (2012).

7. Moraud, E. M. et al. Mechanisms Underlying the Neuromodulation of Spinal Circuits for Correcting Gait and Balance Deficits after Spinal Cord Injury. Neuron 89, 814-828 (2016).

8. Frankemolle, A. M. M. et al. Reversing cognitive-motor impairments in Parkinson's disease patients using a computational modelling approach to deep brain stimulation programming. Brain 133, 746-761 (2010).

9. Aristovich, K. et al. Model-based geometrical optimisation and in vivo validation of a spatially selective multielectrode cuff array for vagus nerve neuromodulation. Journal of Neuroscience Methods 352, 109079 (2021).

10. Rattay, F. Analysis of models for extracellular fiber stimulation. IEEE Trans. Biomed. Eng. 36, 676-682 (1989).

11. Dali, M. et al. Model based optimal multipolar stimulation without a priori knowledge of nerve structure: application to vagus nerve stimulation. J. Neural Eng. 15, 046018 (2018).

12. Delgado-Martinez, I., Badia, J., Pascual-Font, A., Rodriguez-Baeza, A. & Navarro, X. Fascicular Topography of the Human Median Nerve for europrosthetic Surgery. Front. Neurosci. 10, (2016).

13. Valle, G. et al. Biomimetic Intraneural Sensory Feedback Enhances Sensation Naturalness, Tactile Sensitivity, and Manual Dexterity in a Bidirectional Prosthesis. Neuron 100, 37-45.e7 (2018).

14. Raspopovic, S., Capogrosso, M. & Micera, S. A Computational Model for the Stimulation of Rat Sciatic Nerve Using a Transverse Intrafascicular Multichannel Electrode. IEEE Transactions on Neural Systems and Rehabilitation Engineering 19, 333-344 (2011).

15. Veraart, C., Grill, W. M. & Mortimer, J. T. Selective control of muscle activation with a multipolar nerve cuff electrode. IEEE Transactions on Biomedical Engineering 40, 640-653 (1993).

16. Polasek, K. H., Hoyen, H. A., Keith, M. W. & Tyler, D. J. Human Nerve Stimulation Thresholds and Selectivity Using a Multi-contact Nerve Cuff Electrode. IEEE Trans. Neural Syst. Rehabil. Eng. 15, 76-82 (2007).

17. Badi, M. & Wurth, S. Intrafascicular peripheral nerve stimulation produces fine functional hand movements in primates. SCIENCE TRANSLATIONAL MEDICINE 13, 16 (2021).

18. Yoon, D., Biswal, S., Rutt, B., Lutz, A. & Hargreaves, B. Feasibility of 7T MRI for imaging fascicular structures of peripheral nerves: 7T MRI of Peripheral Nerves. Muscle Nerve 57, 494-498 (2018).

19. Cartwright, M. S., Baute, V., Caress, J. B. & Walker, F. O. Ultrahigh-frequency ultrasound of fascicles in the median nerve at the wrist. Muscle Nerve 56, 819-822 (2017).

20. Wodlinger, B. & Durand, D. M. Localization and Recovery of Peripheral Neural Sources With Beamforming Algorithms. IEEE Transactions on Neural Systems and Rehabilitation Engineering 17, 461-468 (2009).

21. Vallone, F. et al. Simultaneous decoding of cardiovascular and respiratory functional changes from pig intraneural vagus nerve signals. J. Neural Eng. 18, 0460a2 (2021).

22. Romeni, S., Valle, G., Mazzoni, A. & Micera, S. Tutorial: a computational framework for the design and optimization of peripheral neural interfaces. Nat Protoc 15, 3129-3153 (2020).

23. Brill, N. A. et al. Evaluation of high-density, multi-contact nerve cuffs for activation of grasp muscles in monkeys. J. Neural Eng. 15, 036003 (2018).

24. Gaillet, V. et al. Spatially selective activation of the visual cortex via intraneural stimulation of the optic nerve. Nat Biomed Eng 4, 181-194 (2020).

25. Oozeer, M., Veraart, C., Legat, V. & Delbeke, J. A model of the mammalian optic nerve fibre based on experimental data. Vision Research 46, 2513-2524 (2006).

26. Joucla, S. & Yvert, B. The "Mirror" Estimate: An Intuitive Predictor of Membrane Polarization during Extracellular Stimulation. Biophysical Journal 96, 3495-3508 (2009).

**Claims**

1. A method for optimizing at least one of a geometry, an implantation procedure, and/or stimulation protocol of one or more electrodes for an electrical stimulation of a target structure in a nervous system of a living being by a computer device, the method comprising the steps of:

    providing a morphology of the target structure;
    performing a geometric optimization of the one or more electrodes based on (i) a geometric parametrization of the one or more electrodes and the morphology of the target structure, and (ii) an objective function evaluating a quality of the one or more electrodes in relationship to the target structure;
    finite element modelling (FEM) of the one or more electrodes implanted in the target structure according to a geometry found during the step of performing the geometric optimization to generate electric potential maps;
    interpolating the electric potential maps to potential fiber node locations of the target structure to build a lead field matrix (LFM);
    biophysical modeling of a stimulation of neural elements populating the neural structure to create a dataset of biophysical model responses based on the LFM and a first subset of potential stimulation protocols;
    fitting responses of a model-based surrogate model or training a machine-learning-based surrogate model of the neural elements of the target structure to the electrical stimulation administered by the one or more electrodes based on the dataset of the biophysical model responses;
    estimating a functional topography of the target structure using a set of recordings or stimulation protocols from the one or more electrodes, the step of estimating being performed after implanting the one or more electrodes;
    performing an optimization of a second subset of the potential stimulation protocols based on (i) the surrogate-model of a response of the neural element to stimulation, (ii) the morphology of the target structure, and (iii) an objective function quantifying an effect of the second subset of potential stimulation protocols to generate a set of candidate optimal stimulation protocols; and
    evaluating the set of candidate optimal stimulation protocols by applying the set of candidate optimal stimulation protocols to the biophysical modeling to determine an optimum stimulation protocol for the target structure.

2. The method of claim 1, further comprising the step of:
    obtaining the morphology of the target structure by at least one of magnetic resonance imaging (MRI) or electrosonography.

3. The method of claim 1, wherein the target structure includes a nerve, and the morphology includes a subdivision of a tridimensional structure of a nerve into a plurality of subregions targeting different functions.

4. The method of claim 3, wherein the subregions targeting different functions include subregions that connect to different muscles, organs, or a combination thereof.

5. The method of claim 3, wherein the morphology includes information on a fascicular morphology of the nerve.

6. The method of claim 1, wherein the target structure includes a brain, and the morphology includes a subdivision of a tridimensional structure of the brain into a plurality of subregions, the subregions performing different functions.

7. The method of claim 1, wherein the target structure includes a spinal cord root, and the morphology includes a subdivision of a tridimensional structure of the spinal cord root into different rootlets.

8. The method of claim 1, wherein the performing the geometric optimization of the one or more electrodes, the objective function includes a second surrogate-model of the FEM of the one or more electrodes and a stimulation protocol optimization.

9. The method of claim 8, wherein the second surrogate-model of the FEM includes an encoder-decoder with a U-Net architecture accepting as input a set of two 3D tensors that define the morphology of the target structure and the first subset of potential stimulation protocols, respectively, and providing an output including a 3D tensor including a value of an extracellular potential at different locations of the target structure.

**10.** The method of claim 1, wherein the step of estimating the functional topography is performed using a functional MRI.

**11.** The method of claim 1, wherein the step of estimating the functional topography is performed using information from recruitment curves obtained by monopolar electrical stimulation.

**12.** The method of claim 1, wherein the step of fitting further includes:
building a surrogate-model including a binary activation classifier that predicts whether neural units are activated by an applied stimulation protocol.

**13.** The method of claim 12, wherein the target structure includes nerve fibers, and the binary activation classifier includes a multilayer perceptron, a support vector machine, a random forest, or a Gaussian process classifier, or a combination thereof, where each of the aforementioned binary activation classifier receive as input data a timely evolution of the extracellular potential appliced to the nerve fibers and a diameter of the nerve fibers.

**14.** The method of claim 12, wherein the target structure includes complex neurons, and the binary activation classifier includes a graph neural network that operates graph classification using a topology of the complex neurons, including information about interconnections between soma, axon, and dendrites, and the extracellular potential applied to the different sections of the complex neurons, and their corresponding morphological features as nodal features.

**15.** The method of claim 14, wherein the step of building the surrogate-model is based on a neural response to an electric stimulation of the one or more electrodes, the surrogate model including a single-output regressor that predicts a firing rate produced in neural units under the applied electric stimulation.

<u>100</u>

FIG. 1

FIG. 2

Segment 1  Segment 2  Segment 3

Block 1

Block 2

Block 3

300

300

300

**FIG. 3A**

Block 1, Segment 1

Section 1 = ref  Section 2  Section 12

301  301  301

$IoU_{ref} = 1$  $IoU_{ref} = 0.8$  $IoU_{ref} = 0.46$

**FIG. 3B**

**A** geometrical parametrization

**B** objective function

minimize average distance

maximize # targeted fascicles

$I_{oc}$

$I_{ss}$

$n_{as}$

d1

d2

d3

d5 d4

x, y, z, θx, θy, θz

**E** lead field matrix

geometrical optimization

FEM 401

400

**D** fiber and node locations

**F** biophysical modelling

generate dataset

402

**C** fascicular topography

**H** functional topography

**G** activation classifier

403 stimulation protocol optimization

404 candidate optima evaluation

405 best stimulation protocol

FIG. 4

FIG. 5A

FIG. 5B

Block 1 : b1
Block 2 : b2
Block 3 : b3

FIG. 5C

FIG. 5D

FIG. 6A

FIG. 6B

FIG. 6C

FIG. 6D

Block 1

Section 1 = ref

Section 2

Section 12

$IoU_{ref} = 1$

$IoU_{ref} = 0.8$

$IoU_{ref} = 0.46$

FIG. 7A

Block 1

| Cluster 1 | Cluster 2 | Cluster 3 | Cluster 4 | Cluster 5 | Cluster 6 |

Section 1: n = 2, n = 2, n = 8, n = 5, n = 8, n = 15

Section 2: n = 3, n = 1, n = 12, n = 7, n = 17

Section 12: n = 6, n = 16, n = 6, n = 2, n = 10

y [mm]

x [mm]

FIG. 7B

FIG. 7C

FIG. 8B

FIG. 8A

FIG. 8C

EP 4 364 790 A1

FIG. 9A

FIG. 9B

FIG. 9C

FIG. 10A

FIG. 10B

FIG. 10C

FIG. 10D

FIG. 10E

EP 4 364 790 A1

| Insertion A | Insertion B | # of occurrences |
|---|---|---|
| 1 | 5 | 2 |
| 2 | 4 | 2 |
| 3 | 4 | 1 |
| **3** | **6** | **7** |
| 4 | 6 | 2 |
| **5** | **6** | **6** |

FIG. 11

| | | Block 1 | Block 2 | Block 3 |
|---|---|---|---|---|
| **m-AMD** | **Monopolar** | 0.997 (562) | 0.990 (756) | 0.985 (1870) |
| | **Tripolar** | 0.964 (3654) | 0.939 (4212) | 0.972 (6256) |
| **M-NPF** | **Monopolar** | 0.984 (1012) | 0.987 (1070) | 0.987 (1664) |
| | **Tripolar** | 0.957 (4592) | 0.973 (4820) | 0.977 (5636) |

FIG. 12

EP 4 364 790 A1

```
-----------------------------------------------------------------
        Layer (type)              Output Shape            Param #
=================================================================
          Conv2d-1              [-1, 32, 64, 64]              608
            ReLU-2              [-1, 32, 64, 64]                0
     BatchNorm2d-3              [-1, 32, 64, 64]               64
          Conv2d-4              [-1, 32, 64, 64]            9,248
            ReLU-5              [-1, 32, 64, 64]                0
     BatchNorm2d-6              [-1, 32, 64, 64]               64
       MaxPool2d-7              [-1, 32, 32, 32]                0
       DownBlock-8   [[-1, 32, 32, 32], [-1, 32, 64, 64]]           0
          Conv2d-9              [-1, 64, 32, 32]           18,496
          ReLU-10              [-1, 64, 32, 32]                0
    BatchNorm2d-11              [-1, 64, 32, 32]              128
         Conv2d-12              [-1, 64, 32, 32]           36,928
          ReLU-13              [-1, 64, 32, 32]                0
    BatchNorm2d-14              [-1, 64, 32, 32]              128
      MaxPool2d-15              [-1, 64, 16, 16]                0
     DownBlock-16   [[-1, 64, 16, 16], [-1, 64, 32, 32]]           0
         Conv2d-17             [-1, 128, 16, 16]           73,856
          ReLU-18             [-1, 128, 16, 16]                0
    BatchNorm2d-19             [-1, 128, 16, 16]              256
         Conv2d-20             [-1, 128, 16, 16]          147,584
          ReLU-21             [-1, 128, 16, 16]                0
    BatchNorm2d-22             [-1, 128, 16, 16]              256
      MaxPool2d-23               [-1, 128, 8, 8]                0
     DownBlock-24   [[-1, 128, 8, 8], [-1, 128, 16, 16]]           0
         Conv2d-25               [-1, 256, 8, 8]          295,168
          ReLU-26               [-1, 256, 8, 8]                0
    BatchNorm2d-27               [-1, 256, 8, 8]              512
         Conv2d-28               [-1, 256, 8, 8]          590,080
          ReLU-29               [-1, 256, 8, 8]                0
    BatchNorm2d-30               [-1, 256, 8, 8]              512
     DownBlock-31   [[-1, 256, 8, 8], [-1, 256, 8, 8]]           0
ConvTranspose2d-32             [-1, 128, 16, 16]          131,200
          ReLU-33             [-1, 128, 16, 16]                0
    BatchNorm2d-34             [-1, 128, 16, 16]              256
    Concatenate-35             [-1, 256, 16, 16]                0
         Conv2d-36             [-1, 128, 16, 16]          295,040
          ReLU-37             [-1, 128, 16, 16]                0
    BatchNorm2d-38             [-1, 128, 16, 16]              256
         Conv2d-39             [-1, 128, 16, 16]          147,584
          ReLU-40             [-1, 128, 16, 16]                0
    BatchNorm2d-41             [-1, 128, 16, 16]              256
      UpBlock-42             [-1, 128, 16, 16]                0
ConvTranspose2d-43              [-1, 64, 32, 32]           32,832
          ReLU-44              [-1, 64, 32, 32]                0
    BatchNorm2d-45              [-1, 64, 32, 32]              128
    Concatenate-46             [-1, 128, 32, 32]                0
         Conv2d-47              [-1, 64, 32, 32]           73,792
          ReLU-48              [-1, 64, 32, 32]                0
    BatchNorm2d-49              [-1, 64, 32, 32]              128
         Conv2d-50              [-1, 64, 32, 32]           36,928
          ReLU-51              [-1, 64, 32, 32]                0
    BatchNorm2d-52              [-1, 64, 32, 32]              128
      UpBlock-53              [-1, 64, 32, 32]                0
ConvTranspose2d-54              [-1, 32, 64, 64]            8,224
          ReLU-55              [-1, 32, 64, 64]                0
    BatchNorm2d-56              [-1, 32, 64, 64]               64
    Concatenate-57              [-1, 64, 64, 64]                0
         Conv2d-58              [-1, 32, 64, 64]           18,464
          ReLU-59              [-1, 32, 64, 64]                0
    BatchNorm2d-60              [-1, 32, 64, 64]               64
         Conv2d-61              [-1, 32, 64, 64]            9,248
    BatchNorm2d-62              [-1, 32, 64, 64]               64
      UpBlock-63              [-1, 32, 64, 64]                0
         Conv2d-64               [-1, 1, 64, 64]               33
=================================================================
Total params: 1,928,577
Trainable params: 1,928,577
Non-trainable params: 0
-----------------------------------------------------------------
Input size (MB): 0.03
Forward/backward pass size (MB): 12256.28
Params size (MB): 7.36
Estimated Total Size (MB): 12263.67
-----------------------------------------------------------------
```

FIG. 13

1400      1401      1402      1403

FIG. 14

FIG. 15

EP 4 364 790 A1

| Layer (type) | Output Shape | Param # |
|---|---|---|
| conv2d (Conv2D) | (None, 71, 67, 32) | 320 |
| batch_normalization (BatchN ormalization) | (None, 71, 67, 32) | 128 |
| conv2d_1 (Conv2D) | (None, 71, 67, 32) | 9248 |
| batch_normalization_1 (Batc hNormalization) | (None, 71, 67, 32) | 128 |
| max_pooling2d (MaxPooling2D ) | (None, 35, 33, 32) | 0 |
| dropout (Dropout) | (None, 35, 33, 32) | 0 |
| conv2d_2 (Conv2D) | (None, 35, 33, 64) | 18496 |
| batch_normalization_2 (Batc hNormalization) | (None, 35, 33, 64) | 256 |
| conv2d_3 (Conv2D) | (None, 35, 33, 64) | 36928 |
| batch_normalization_3 (Batc hNormalization) | (None, 35, 33, 64) | 256 |
| max_pooling2d_1 (MaxPooling 2D) | (None, 17, 16, 64) | 0 |
| dropout_1 (Dropout) | (None, 17, 16, 64) | 0 |
| conv2d_4 (Conv2D) | (None, 17, 16, 128) | 73856 |
| batch_normalization_4 (Batc hNormalization) | (None, 17, 16, 128) | 512 |
| conv2d_5 (Conv2D) | (None, 17, 16, 128) | 147584 |
| batch_normalization_5 (Batc hNormalization) | (None, 17, 16, 128) | 512 |
| max_pooling2d_2 (MaxPooling 2D) | (None, 8, 8, 128) | 0 |
| dropout_2 (Dropout) | (None, 8, 8, 128) | 0 |
| flatten (Flatten) | (None, 8192) | 0 |
| dense (Dense) | (None, 64) | 524352 |
| batch_normalization_6 (Batc hNormalization) | (None, 64) | 256 |
| dropout_3 (Dropout) | (None, 64) | 0 |
| dense_1 (Dense) | (None, 32) | 2080 |
| batch_normalization_7 (Batc hNormalization) | (None, 32) | 128 |
| dropout_4 (Dropout) | (None, 32) | 0 |
| dense_2 (Dense) | (None, 2) | 66 |

Total params: 815,106
Trainable params: 814,018
Non-trainable params: 1,088

FIG. 16

FIG. 17

FIG. 18A

FIG. 18B

EP 4 364 790 A1

FIG. 18E

FIG. 18D

FIG. 18C

FIG. 19A

FIG. 19B

EP 4 364 790 A1

## Group 3

## Group 4

| -0.02 | 0 | 0 | 0 | 0 | 0 | 0 |
| ▲ | ▲ | ▲ | ▲ | ▲ | ▲ | ▲ |
| ▲ | ▲ | ▲ | ▲ | ▲ | ▲ | ▲ |
| 0 | 0.04 | 0 | 0 | -0.04 | 0 | 0 |

| 0.05 | -0.01 | 0 | -0.02 | 0 | 0 | 0 |
| ▲ | ▲ | ▲ | ▲ | ▲ | ▲ | ▲ |
| ▲ | ▲ | ▲ | ▲ | ▲ | ▲ | ▲ |
| 0 | 0 | 0 | 0 | 0 | 0 | 0 |

FIG. 19C

FIG. 19D

Group 5

FIG. 19E

Europäisches
Patentamt

European
Patent Office

Office européen
des brevets

# EUROPEAN SEARCH REPORT

Application Number

EP 22 20 4926

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/104479 A1 (GRILL WARREN M [US] ET AL) 19 April 2018 (2018-04-19)<br>* the whole document * | 1-15 | INV.<br>A61N1/36 |
| A | US 2012/330622 A1 (BUTSON CHRISTOPHER R [US] ET AL) 27 December 2012 (2012-12-27)<br>* the whole document * | 1-15 | |
| A | WO 2011/025865 A1 (CLEVELAND CLINIC FOUNDATION [US]; LUJAN J LUIS [US] ET AL.) 3 March 2011 (2011-03-03)<br>* the whole document * | 1-15 | |
| A | CN 115 105 743 A (SHANGHAI LISTENT MEDICAL TECH CO LTD) 27 September 2022 (2022-09-27)<br>* the whole document * | 1-15 | |

TECHNICAL FIELDS
SEARCHED       (IPC)

A61N

The present search report has been drawn up for all claims

| Place of search | Date of completion of the search | Examiner |
|---|---|---|
| Munich | 16 March 2023 | Molina Silvestre, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 4926

This annex lists the patent family members relating to the patent documents cited in the above-mentioned European search report.
The members are as contained in the European Patent Office EDP file on
The European Patent Office is in no way liable for these particulars which are merely given for the purpose of information.

16-03-2023

| Patent document cited in search report | | Publication date | Patent family member(s) | | Publication date |
|---|---|---|---|---|---|
| US 2018104479 | A1 | 19-04-2018 | US 2018104479 A1 | | 19-04-2018 |
| | | | WO 2016209682 A1 | | 29-12-2016 |
| US 2012330622 | A1 | 27-12-2012 | US 2007083104 A1 | | 12-04-2007 |
| | | | US 2012330622 A1 | | 27-12-2012 |
| WO 2011025865 | A1 | 03-03-2011 | AU 2010286603 A1 | | 22-03-2012 |
| | | | CA 2772330 A1 | | 03-03-2011 |
| | | | EP 2470258 A1 | | 04-07-2012 |
| | | | JP 5734295 B2 | | 17-06-2015 |
| | | | JP 2013502999 A | | 31-01-2013 |
| | | | US 2011191275 A1 | | 04-08-2011 |
| | | | US 2013218819 A1 | | 22-08-2013 |
| | | | US 2019287020 A1 | | 19-09-2019 |
| | | | US 2021220656 A1 | | 22-07-2021 |
| | | | WO 2011025865 A1 | | 03-03-2011 |
| CN 115105743 | A | 27-09-2022 | NONE | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- A Computer-Brain Interface that Restores Lost Extremities' Touch and Movement Sensations. **VALLE, G. ; PETRINI, F. M. ; MIJOVIC, P. ; MIJOVIC, B. ; RASPOPOVIC, S.** Brain-Computer Interface Research. Springer International Publishing, 2021, 65-73
- **ROWALD, A. et al.** Activity-dependent spinal cord neuromodulation rapidly restores trunk and leg motor functions after complete paralysis. *Nat Med,* 2022, vol. 28, 260-271
- **BENABID, A. L.** Deep brain stimulation for Parkinson's disease. *Current Opinion in Neurobiology,* 2003, vol. 13, 696-706
- **RASPOPOVIC, S. ; CAPOGROSSO, M. ; BADIA, J. ; NAVARRO, X. ; MICERA, S.** Experimental Validation of a Hybrid Computational Model for Selective Stimulation Using Transverse Intrafascicular Multichannel Electrodes. *IEEE Transactions on Neural Systems and Rehabilitation Engineering,* 2012, vol. 20, 395-404
- **MORAUD, E. M. et al.** Mechanisms Underlying the Neuromodulation of Spinal Circuits for Correcting Gait and Balance Deficits after Spinal Cord Injury. *Neuron,* 2016, vol. 89, 814-828
- **FRANKEMOLLE, A. M. M. et al.** Reversing cognitive-motor impairments in Parkinson's disease patients using a computational modelling approach to deep brain stimulation programming. *Brain,* 2010, vol. 133, 746-761
- **ARISTOVICH, K. et al.** Model-based geometrical optimisation and in vivo validation of a spatially selective multielectrode cuff array for vagus nerve neuromodulation. *Journal of Neuroscience Methods,* 2021, vol. 352, 109079
- **RATTAY, F.** Analysis of models for extracellular fiber stimulation. *IEEE Trans. Biomed. Eng.,* 1989, vol. 36, 676-682
- **DALI, M. et al.** Model based optimal multipolar stimulation without a priori knowledge of nerve structure: application to vagus nerve stimulation. *J. Neural Eng.,* 2018, vol. 15, 046018
- **DELGADO-MARTINEZ, I. ; BADIA, J. ; PASCUAL-FONT, A. ; RODRIGUEZ-BAEZA, A. ; NAVARRO, X.** Fascicular Topography of the Human Median Nerve for europrosthetic Surgery. *Front. Neurosci.,* 2016, vol. 10

- **VALLE, G. et al.** Biomimetic Intraneural Sensory Feedback Enhances Sensation Naturalness, Tactile Sensitivity, and Manual Dexterity in a Bidirectional Prosthesis. *Neuron,* 2018, vol. 100, 37-45.e7
- **RASPOPOVIC, S. ; CAPOGROSSO, M. ; MICERA, S.** A Computational Model for the Stimulation of Rat Sciatic Nerve Using a Transverse Intrafascicular Multichannel Electrode. *IEEE Transactions on Neural Systems and Rehabilitation Engineering,* 2011, vol. 19, 333-344
- **VERAART, C. ; GRILL, W. M. ; MORTIMER, J. T.** Selective control of muscle activation with a multipolar nerve cuff electrode. *IEEE Transactions on Biomedical Engineering,* 1993, vol. 40, 640-653
- **POLASEK, K. H. ; HOYEN, H. A. ; KEITH, M. W. ; TYLER, D. J.** Human Nerve Stimulation Thresholds and Selectivity Using a Multi-contact Nerve Cuff Electrode. *IEEE Trans. Neural Syst. Rehabil. Eng.,* 2007, vol. 15, 76-82
- **BADI, M. ; WURTH, S.** Intrafascicular peripheral nerve stimulation produces fine functional hand movements in primates. *SCIENCE TRANSLATIONAL MEDICINE,* 2021, vol. 13, 16
- **YOON, D. ; BISWAL, S. ; RUTT, B. ; LUTZ, A. ; HARGREAVES, B.** Feasibility of 7T MRI for imaging fascicular structures of peripheral nerves: 7T MRI of Peripheral Nerves. *Muscle Nerve,* 2018, vol. 57, 494-498
- **CARTWRIGHT, M. S. ; BAUTE, V. ; CARESS, J. B. ; WALKER, F. O.** Ultrahigh-frequency ultrasound of fascicles in the median nerve at the wrist. *Muscle Nerve,* 2017, vol. 56, 819-822
- **WODLINGER, B. ; DURAND, D. M.** Localization and Recovery of Peripheral Neural Sources With Beamforming Algorithms. *IEEE Transactions on Neural Systems and Rehabilitation Engineering,* 2009, vol. 17, 461-468
- **VALLONE, F. et al.** Simultaneous decoding of cardiovascular and respiratory functional changes from pig intraneural vagus nerve signals. *J. Neural Eng.,* 2021, vol. 18, 0460a2
- **ROMENI, S. ; VALLE, G ; MAZZONI, A. ; MICERA, S.** Tutorial: a computational framework for the design and optimization of peripheral neural interfaces. *Nat Protoc,* 2020, vol. 15, 3129-3153
- **BRILL, N. A. et al.** Evaluation of high-density, multi-contact nerve cuffs for activation of grasp muscles in monkeys. *J. Neural Eng.,* 2018, vol. 15, 036003

- **GAILLET, V. et al.** Spatially selective activation of the visual cortex via intraneural stimulation of the optic nerve. *Nat Biomed Eng,* 2020, vol. 4, 181-194
- **OOZEER, M. ; VERAART, C. ; LEGAT, V. ; DELBEKE, J.** A model of the mammalian optic nerve fibre based on experimental data. *Vision Research,* 2006, vol. 46, 2513-2524
- **JOUCLA, S. ; YVERT, B.** The "Mirror" Estimate: An Intuitive Predictor of Membrane Polarization during Extracellular Stimulation. *Biophysical Journal,* 2009, vol. 96, 3495-3508